# EUROPEAN PATENT SPECIFICATION

(11) **EP 1 670 932 B1**
(45) Date of publication and mention of the grant of the patent: **30.03.2011**
(21) Application number: 04786588.6
(22) Date of filing: 26.08.2004
(51) Int. Cl.: C12N 15/10, C12Q 1/68

(54) **LIBRARIES OF RECOMBINANT CHIMERIC PROTEINS**
BIBLIOTHEKEN REKOMBINANTER CHIMÄRISCHER PROTEINE
BANQUES DE GENES DE PROTEINES CHIMERES RECOMBINANTES

(30) Priority: 27.08.2003 US 497924 P; 26.08.2004 US 926542
(43) Date of publication of application: 21.06.2006
(60) Divisional application: 10180571.1
(73) Proprietor: Proterec Ltd, Ramat-Gan 52656 (IL)
(72) Inventor: SHARON, Gil, Mevaseret Zion (IL); LABAN, Abraham, Jerusalem (IL)
(74) Representative: Andrews, Martyn Peter
(86) International application number: PCT/US2004/027793
(87) International publication number: WO 2005/021719

(56) References cited:
- WO-A-00/43507
- WO-A-01/75091
- WO-A-98/32845
- WO-A-03/044198
- WO-A-2006/047669
- US-A- 6 107 059
- US-A- 6 117 679
- US-B1- 6 506 603
- SODERLIND E ET AL: "RECOMBINING GERMLINE-DERIVED CDR SEQUENCES FOR CREATING DIVERSE SINGLE-FRAMEWORK ANTIBODY LIBRARIES" NATURE BIOTECHNOLOGY, NATURE PUBLISHING GROUP, NEW YORK, NY, US, vol. 18, no. 8, August 2000 (2000-08), pages 852-856, XP009010618 ISSN: 1087-0156
- GIBBS M D ET AL: "Degenerate oligonucleotide gene shuffling (DOGS): a method for enhancing the frequency of recombination with family shuffling" GENE, ELSEVIER, AMSTERDAM, NL, vol. 271, no. 1, 13 June 2001 (2001-06-13), pages 13-20, XP004245489 ISSN: 0378-1119
- KNAPPIK A ET AL: "Fully synthetic human combinatorial antibody libraries (HuCAL) based on modular consensus frameworks and CDRs randomized with trinucleotides" JOURNAL OF MOLECULAR BIOLOGY, LONDON, GB, vol. 296, no. 1, 11 February 2000 (2000-02-11), pages 57-86, XP004461525 ISSN: 0022-2836
- JIRHOLT P ET AL: "A central core structure in an antibody variable domain determines antigen specificity." PROTEIN ENGINEERING JAN 2001, vol. 14, no. 1, January 2001 (2001-01), pages 67-74, XP002475988 ISSN: 0269-2139
- JIRHOLT P ET AL: "Exploiting sequence space: shuffling in vivo formed complementarity determining regions into a master framework" GENE, ELSEVIER, AMSTERDAM, NL, vol. 215, no. 2, July 1998 (1998-07), pages 471-476, XP004149272 ISSN: 0378-1119
- ZHA DONGXING ET AL: "Assembly of designed oligonucleotides as an efficient method for gene recombination: a new tool in directed evolution." CHEMBIOCHEM : A EUROPEAN JOURNAL OF CHEMICAL BIOLOGY 3 JAN 2003, vol. 4, no. 1, 3 January 2003 (2003-01-03) , pages 34-39, ISSN: 1439-4227

### SEQUENCE LISTING

<110> PROTEREC LTD.
   LABAN, ABRAHAM
   SHARON, GIL
<120> LIBRARIES OF RECOMBINANT PROTEINS
<130> 178.002
<140>
   <141>
<150> US 60/497,924
   <151> 2003-08-27
<160> 127
<170> Patent In Ver. 3.2
<210> 1
   <211> 43
   <212> DNA
   <213> Artificial Sequence
<220>
   <223> Description of Combined DNA/RNA Molecule: Primer
<220>
   <223> Description of Artificial Sequence: Primer
<400> 1
   aagggguacc tttggauaaa agactggacc gacgggcggc gct 43
<210> 2
   <211> 42
   <212> DNA
   <213> Artificial Sequence
<220>
   <223> Description of Combined DNA/RNA Molecule: Primer
<220>
   <223> Description of Artificial Sequence: Primer
<400> 2
   aagaagucca aagcttcagc uctaaggtgg tgtgatgggg cc 42
<210> 3
   <211> 43
   <212> DNA
   <213> Artificial Sequence
<220>
   <223> Description of Combined DNA/RNA Molecule: Primer
<220>
   <223> Description of Artificial Sequence: Primer
<400> 3
   aagggguacc tttggauaaa agatccccct tgactgtaaa gtc 43
<210> 4
   <211> 42
   <212> DNA
   <213> Artificial Sequence
<220>
   <223> Description of Combined DNA/RNA.Molecule: Primer
<220>
   <223> Description of Artificial Sequence: Primer
<400> 4
   aagaagucca aagcttcagc uctattttaa gtcaataaag tt 42
<210> 5
   <211> 43
   <212> DNA
   <213> Artificial Sequence
<220>
   <223> Description of Combined DNA/RNA Molecule: Primer
<220>
   <223> Description of Artificial Sequence: Primer
<400> 5
   aagggguacc tttggauaaa agaggtctta ttttaccaac taa 43
<210> 6
   <211> 42
   <212> DNA
   <213> Artificial Sequence
<220>
   <223> Description of Combined DNA/RNA Molecule: Primer
<220>
   <223> Description of Artificial Sequence: Primer
<400> 6
   aagaagucca aagcttcagc uctatacaag tattgaaatc tt 42
<210> 7
   <211> 43
   <212> DNA
   <213> Artificial Sequence
<220>
   <223> Description of Combined DNA/RNA Molecule: Primer
<220>
   <223> Description of Artificial Sequence: Primer
<400> 7
   aagggguacc tttggauaaa agagccgtca ttgccccagt taa 43
<210> 8
   <211> 42
   <212> DNA
   <213> Artificial Sequence
<220>
   <223> Description of Combined DNA/RNA Molecule: Primer
<220>
   <223> Description of Artificial Sequence: Primer
<400> 8
   aagaagucca aagcttcagc uttacacttt tacatactct aa 42
<210> 9
   <211> 23
   <212> DNA
   <213> Artificial Sequence
<220>
   <223> Description of Combined DNA/RNA Molecule: Primer
<220>
   <223> Description of Artificial Sequence: Primer
<400> 9
   atccaaaggu acccctuctt ctt 23
<210> 10
   <211> 23
   <212> DNA
   <213> Artificial Sequence
<220>
   <223> Description of Combined DNA/RNA Molecule: Primer
<220>
   <223> Description of Artificial Sequence: Primer
<400> 10
   agctgaagct utggacttct ucg 23
<210> 11
   <211> 42
   <212> DNA
   <213> Artificial Sequence
<220>
   <223> Description of Combined DNA/RNA Molecule: Primer
<220>
   <223> Description of Artificial Sequence: Primer
<400> 11
   atcctcguag acctggtaac uaaagatctt gggcggcgaa gc 42
<210> 12
   <211> 42
   <212> DNA
   <213> Artificial Sequence
<220>
   <223> Description of Combined DNA/RNA Molecule: Primer
<220>
   <223> Description of Artificial Sequence: Primer
<400> 12
   atcctcguag acctggtaac uaaggacttt ggatgggtca gc 42
<210> 13
   <211> 42
   <212> DNA
   <213> Artificial Sequence
<220>
   <223> Description of Combined DNA/RNA Molecule: Primer
<220>
   <223> Description of Artificial Sequence: Primer
<400> 13
   atcctcguag acctggtaac uagcgagttt ggaagggtca gc 42
<210> 14
   <211> 42
   <212> DNA
   <213> Artificial Sequence
<220>
   <223> Description of Combined DNA/RNA Molecule: Primer
<220>
   <223> Description of Artificial Sequence: Primer
<400> 14
   atcctcguag acctggtaac ucaccacctt gtcagaagtt gc 42
<210> 15
   <211> 44
   <212> DNA
   <213> Artificial Sequence
<220>
   <223> Description of Combined DNA/RNA Molecule: Primer
<220>
   <223> Description of Artificial Sequence: Primer
<400> 15
   agttaccagg uctacgagga ugccacggcg ctcgactgtg ctcc 44
<210> 16
   <211> 44
   <222> DNA
   <213> Artificial Sequence
<220>
   <223> Description of Combined DNA/RNA Molecule: Primer
<220>
   <223> Description of Artificial Sequence: Primer
<400> 16
   agttaccagg uctacgagga utctgcaaat attgaatgtt cacc 44
<210> 17
   <211> 44
   <212> DNA
   <213> Artificial Sequence
<220>
   <223> Description of Combined DNA/RNA Molecule: Primer
<220>
   <223> Description of Artificial Sequence: Primer
<400> 17
   agttaccagg uctacgagga utccgctaaa gctgattgtg cccc 44
<210> 18
   <211> 44
   <212> DNA
   <213> Artificial Sequence
<220>
   <223> Description of Combined DNA/RNA Molecule: Primer
<220>
   <223> Description of Artificial Sequence: Primer
<400> 18
   agttaccagg uctacgagga ugcagcaaac ttggattttt cacc 44
<210> 19
   <211> 44
   <212> DNA
   <213> Artificial Sequence
<220>
   <223> Description of Combined DNA/RNA Molecule: Primer
<220>
   <223> Description of Artificial Sequence: Primer
<400> 19
   agagacgacg uagtagcccu gctgcagcgc ccagccgatg atga 44
<210> 20
   <211> 44
   <212> DNA
   <213> Artificial Sequence
<220>
   <223> Description of Combined DNA/RNA Molecule: Primer
<220>
   <223> Description of Artificial Sequence: Primer
<400> 20
   agagacgacg uagtagcccu gcttcaacat aggtaccatt aaag 44
<210> 21
   <211> 44
   <212> DNA
   <213> Artificial Sequence
<220>
   <223> Description of Combined DNA/RNA Molecule: Primer
<220>
   <223> Description of Artificial Sequence: Primer
<400> 21
   agagacgacg uagtagcccu ggtccaataa tggtgccaat aaat 44
<210> 22
   <211> 44
   <212> DNA
   <213> Artificial Sequence
<220>
   <223> Description of Combined DNA/RNA Molecule: Primer
<220>
   <223> Description of Artificial Sequence: Primer
<400> 22
   agagacgacg uagtagcccu gatccaacaa tgctgacata aaat 44
<210> 23
   <211> 40
   <212> DNA
   <213> Artificial Sequence
<220>
   <223> Description of Combined DNA/RNA Molecule: Primer
<220>
   <223> Description of Artificial Sequence: Primer
<400> 23
   agggctacua cgtcgtctcu tccgaccacg aaggcttcaa 40
<210> 24
   <211> 40
   <212> DNA
   <213> Artificial Sequence
<220>
   <223> Description of Combined DNA/RNA Molecule: Primer
<220>
   <223> Description of Artificial Sequence: Primer
<400> 24
   agggctacua cgtcgtctcu cctgattatg agggaccaaa 40
<210> 25
   <211> 40
   <212> DNA
   <213> Artificial Sequence
<220>
   <223> Description of Combined DNA/RNA Molecule: Primer
<220>
   <223> Description of Artificial Sequence: Primer
<400> 25
   agggctacua cgtcgtctcu ccagactatg aaggtcctaa 40
<210> 26
   <211> 40
   <212> DNA
   <213> Artificial Sequence
<220>
   <223> Description of Combined DNA/RNA Molecule: Primer
<220>
   <223> Description of Artificial Sequence: Primer
<400> 26
   agggctacua cgtcgtctcu cccgattacg aaggtccaaa 40
<210> 27
   <211> 42
   <212> DNA
   <213> Artificial Sequence
<220>
   <223> Description of Combined DNA/RNA Molecule: Primer
<220>
   <223> Description of Artificial Sequence: Primer
<400> 27
   acctccgugc gaagctccua caatgttgag ctcgggcgcg ta 42
<210> 28
   <211> 42
   <212> DNA
   <213> Artificial Sequence
<220>
   <223> Description of Combined DNA/RNA Molecule: Primer
<220>
   <223> Description of Artificial Sequence: Primer
<400> 28
   acctccgugc gaagctccua ccaaattttt cttcaattct gg 42
<210> 29
   <211> 42
   <212> DNA
   <213> Artificial Sequence
<220>
   <223> Description of Combined DNA/RNA Molecule: Primer
<220>
   <223> Description of Artificial Sequence: Primer
<400> 29
   acctccgugc gaagctccua ctaagttacc acccaattct gg 42
<210> 30
   <211> 42
   <212> DNA
   <213> Artificial Sequence
<220>
   <223> Description of Combined DNA/RNA Molecule: Primer
<220>
   <223> Description of Artificial Sequence: Primer
<400> 30
   acctccgugc gaagctccua ccaaattgtc agtcaattct gg 42
<210> 31
   <211> 40
   <212> DNA
   <213> Artificial Sequence
<220>
   <223> Description of Combined DNA/RNA Molecule: Primer
<220>
   <223> Description of Artificial Sequence: Primer
<400> 31
   aggagctucg cacggaggua cgccagtgag cgccaaggac 40
<210> 32
   <211> 40
   <212> DNA
   <213> Artificial Sequence
<220>
   <223> Description of Combined DNA/RNA Molecule: Primer
<220>
   <223> Description of Artificial Sequence: Primer
<400> 32
   aggagctucg cacggaggut ttgtcactaa tattactgcc 40
<210> 33
   <211> 40
   <212> DNA
   <213> Artificial Sequence
<220>
   <223> Description of Combined DNA/RNA Molecule: Primer
<220>
   <223> Description of Artificial Sequence: Primer
<400> 33
   aggagctucg cacggaggut ttgttactaa tattactgct 40
<210> 34
   <211> 40
   <212> DNA
   <213> Artificial Sequence
<220>
   <223> Description of Combined DNA/RNA Molecule: Primer
<220>
   <223> Description of Artificial Sequence: Primer
<400> 34
   aggagctucg cacggaggut ttgtcacaaa tataacggct 40
<210> 35
   <211> 47
   <212> DNA
   <213> Artificial Sequence
<220>
   <223> Description of Combined DNA/RNA Molecule: Primer
<220>
   <223> Description of Artificial Sequence: Primer
<400> 35
   atgccagaug aagcgcggga acutgggcac cgataccgtg tagctcg 47
<210> 36
   <211> 47
   <212> DNA
   <213> Artificial Sequence
<220>
   <223> Description of Combined DNA/RNA Molecule: Primer
<220>
   <223> Description of Artificial Sequence: Primer
<400> 36
   atgccagaug aagcgcggga acutgggtaa ataatcttta tccattt 47
<210> 37
   <211> 47
   <212> DNA
   <213> Artificial Sequence
<220>
   <223> Description of Combined DNA/RNA Molecule: Primer
<220>
   <223> Description of Artificial Sequence: Primer
<400> 37
   atgccagaug aagcgcggga acutgggaac tagctgtttc tgataaa 47
<210> 38
   <211> 47
   <212> DNA
   <213> Artificial Sequence
<220>
   <223> Description of Combined DNA/RNA Molecule: Primer
<220>
   <223> Description of Artificial Sequence: Primer
<400> 38
   atgccagaug aagcgcggga acutgggaag atattttttc ggttgat 47
<210> 39
   <211> 43
   <212> DNA
   <213> Artificial Sequence
<220>
   <223> Description of Combined DNA/RNA Molecule: Primer
<220>
   <223> Description of Artificial Sequence: Primer
<400> 39
   agttcccgcg cutcatctgg caugcgatcc ccgacgagat cgt 43
<210> 40
   <211> 43
   <212> DNA
   <213> Artificial Sequence
<220>
   <223> Description of Combined DNA/RNA Molecule: Primer
<220>
   <223> Description of Artificial Sequence: Primer
<400> 40
   agttcccgcg cutcatctgg cauggtgcat tggattccat tgt 43
<210> 41
   <211> 43
   <212> DNA
   <213> Artificial Sequence
<220>
   <223> Description of Combined DNA/RNA Molecule: Primer
<220>
   <223> Description of Artificial Sequence: Primer
<400> 41
   agttcccgcg cutcatctgg cauggtgcta ttgaccaaat tgt 43
<210> 42
   <211> 43
   <212> DNA
   <213> Artificial Sequence
<220>
   <223> Description of Combined DNA/RNA Molecule: Primer
<220>
   <223> Description of Artificial Sequence: Primer
<400> 42
   agttcccgcg cutcatctgg cauggaactc aggataatat tgt 43
<210> 43
   <211> 42
   <212> DNA
   <213> Artificial Sequence
<220>
   <223> Description of Combined DNA/RNA Molecule: Primer
<220>
   <223> Description of Artificial Sequence: Primer
<400> 43
   aggcttgctu gataaaccau aagctaggca ccagaccaaa ga 42
<210> 44
   <211> 42
   <212> DNA
   <213> Artificial Sequence
<220>
   <223> Description of Combined DNA/RNA Molecule: Primer
<220>
   <223> Description of Artificial Sequence: Primer
<400> 44
   aggcttgctu gataaaccau aaagctgccg gggccccaac aa 42
<210> 45
   <211> 42
   <212> DNA
   <213> Artificial Sequence
<220>
   <223> Description of Combined DNA/RNA Molecule: Primer
<220>
   <223> Description of Artificial Sequence: Primer
<400> 45
   aggcttgctu gataaaccau aaagcaacag gagcgccaac aa 42
<210> 46
   <211> 42
   <212> DNA
   <213> Artificial Sequence
<220>
   <223> Description of Combined DNA/RNA Molecule: Primer
<220>
   <223> Description of Artificial Sequence: Primer
<400> 46
   aggcttgctu gataaaccau aaagcagccg gcgccccgac aa 42
<210> 47
   <211> 42
   <212> DNA
   <213> Artificial Sequence
<220>
   <223> Description of Combined DNA/RNA Molecule: Primer
<220>
   <223> Description of Artificial Sequence: Primer
<400> 47
   atggtttauc aagcaagccu tcgacggcac cacacccaag gt 42
<210> 48
   <211> 42
   <212> DNA
   <213> Artificial Sequence
<220>
   <223> Description of Combined DNA/RNA Molecule: Primer
<220>
   <223> Description of Artificial Sequence: Primer
<400> 48
   atggtttauc aagcaagccu tcgacggtga accagtcgtc aa 42
<210> 49
   <211> 42
   <212> DNA
   <213> Artificial Sequence
<220>
   <223> Description of Combined DNA/RNA Molecule: Primer
<220>
   <223> Description of Artificial Sequence: Primer
<400> 49
   atggtttauc aagcaagccu tcaatggaaa acaaaccgtg tc 42
<210> 50
   <211> 42
   <212> DNA
   <213> Artificial Sequence
<220>
   <223> Description of Combined DNA/RNA Molecule: Primer
<220>
   <223> Description of Artificial Sequence: Primer
<400> 50
   atggtttauc aagcaagccu tcaatggagt tgagcctgtt ca 42
<210> 51
   <211> 24
   <212> DNA
   <213> Artificial Sequence
<220>
   <223> Description of Artificial Sequence: Synthetic oligonucleotide
<400> 51
   atgttctttc ctgcgttcag gcgt 24
<210> 52
   <211> 22
   <212> DNA
   <213> Artificial Sequence
<220>
   <223> Description of Artificial Sequence: Synthetic oligonucleotide
<400> 52
   aatcagctag tgactggacg at 22
<210> 53
   <211> 24
   <212> DNA
   <213> Artificial Sequence
<220>
   <223> Description of Artificial Sequence: Synthetic oligonucleotide
<400> 53
   ttgacagctt atcatcgata agct 24
<210> 54
   <211> 31
   <212> DNA
   <213> Artificial Sequence
<220>
   <223> Description of Artificial Sequence: Synthetic oligonucleotide
<400> 54
   caagacgtag cccagcgcgt cggccggcga t 31
<210> 55
   <211> 42
   <212> DNA
   <213> Artificial Sequence
<220>
   <223> Description of Combined DNA/RNA Molecule: Primer
<220>
   <223> Description of Artificial Sequence: Primer
<400> 55
   ttgacagcut atcaucgata agcutgctac ttggagccac ta 42
<210> 56
   <211> 44
   <212> DNA
   <213> Artificial Sequence
<220>
   <223> Description of Combined DNA/RNA Molecule: Primer
<220>
   <223> Description of Artificial Sequence: Primer
<400> 56
   acgccugaac gcaggaaaga acauggatcc acaggacggg tgtg 44
<210> 57
   <211> 47
   <212> DNA
   <213> Artificial Sequence
<220>
   <223> Description of Combined DNA/RNA Molecule: Primer
<220>
   <223> Description of Artificial Sequence: Primer
<400> 57
   ttgacagcut atcaucgata agcutcgggt gtcggggcgc agccatg 47
<210> 58
   <211> 44
   <212> DNA
   <213> Artificial Sequence
<220>
   <223> Description of Combined DNA/RNA Molecule: Primer
<220>
   <223> Description of Artificial Sequence: Primer
<400> 58
   acgccugaac gcaggaaaga acauaccgca tatggtgcac tctc 44
<210> 59
   <211> 44
   <212> DNA
   <213> Artificial Sequence
<220>
   <223> Description of Combined DNA/RNA Molecule: Primer
<220>
   <223> Description of Artificial Sequence: Primer
<400> 59
   atgttctutc ctgcgutcag gcgutttcgg cgtgggtatg gtgg 44
<210> 60
   <211> 45
   <212> DNA
   <213> Artificial Sequence
<220>
   <223> Description of Combined DNA/RNA Molecule: Primer
<220>
   <223> Description of Artificial Sequence: Primer
<400> 60
   aatcagcuag tgacuggacg auatcggtcg acgctctccc ttatg 45
<210> 61
   <211> 47
   <212> DNA
   <213> Artificial Sequence
<220>
   <223> Description of Combined DNA/RNA Molecule: Primer
<220>
   <223> Description of Artificial Sequence: Primer
<400> 61
   atgttctutc ctgcgutcag gcgucggatt caccactcca agaattg 47
<210> 62
   <211> 42
   <212> DNA
   <213> Artificial Sequence
<220>
   <223> Description of Combined DNA/RNA Molecule: Primer
<220>
   <223> Description of Artificial Sequence: Primer
<400> 62
   aatcagcuag tgacuggacg augcgcattc acagttctcc gc 42
<210> 63
   <211> 43
   <212> DNA
   <213> Artificial Sequence
<220>
   <223> Description of Combined DNA/RNA Molecule: Primer
<220>
   <223> Description of Artificial Sequence: Primer
<400> 63
   atcguccagt cacuagctga tugaaaaaac cgcccttaac atg 43
<210> 64
   <211> 46
   <212> DNA
   <213> Artificial Sequence
<220>
   <223> Description of Combined DNA/RNA Molecule: Primer
<220>
   <223> Description of Artificial Sequence: Primer
<400> 64
   caagacguag cccagcgcgu cggccggcga utcacagatg tctgcc 46
<210> 65
   <211> 44
   <212> DNA
   <213> Artificial Sequence
<220>
   <223> Description of Combined DNA/RNA Molecule: Primer
<220>
   <223> Description of Artificial Sequence: Primer
<400> 65
   atcguccagt cacuagctga tugactcccc gtcgtgtaga taac 44
<210> 66
   <211> 52
   <212> DNA
   <213> Artificial Sequence
<220>
   <223> Description of Combined DNA/RNA Molecule: Primer
<220>
   <223> Description of Artificial Sequence: Primer
<400> 66
   caagacguag cccagcgcgu cggccggcga ugttgggaac cggagctgaa tg 52
<210> 67
   <211> 57
   <212> DNA
   <213> Artificial Sequence
<220>
   <223> Description of Combined DNA/RNA Molecule: Primer
<220>
   <223> Description of Artificial Sequence: Primer
<400> 67
   atcgccggcc gacgcgcugg gcuacgtctt gcauatgcgg tgtgaaatac cgcacag 57
<210> 68
   <211> 46
   <212> DNA
   <213> Artificial Sequence
<220>
   <223> Description of Combined DNA/RNA Molecule: Primer
<220>
   <223> Description of Artificial Sequence: Primer
<400> 68
   gcttatcgat gauaagctgu caaugagaca ataaccctga taaatg 46
<210> 69
   <211> 20
   <212> DNA
   <213> Artificial Sequence
<220>
   <223> Description of Artificial Sequence: Primer
<400> 69
   agggcgacac ggaaatgttg 20
<210> 70
   <211> 20
   <212> DNA
   <213> Artificial Sequence
<220>
   <223> Description of Artificial Sequence: Primer
<400> 70
   aagcggaaga gcgcctgatg 20
<210> 71
   <211> 60
   <212> DNA
   <213> Artificial Sequence
<220>
   <223> Description of Combined DNA/RNA Molecule: Primer
<220>
   <223> Description of Artificial Sequence: Primer
<400> 71
   aaagtggtcg gutcttattu cgtcgagtgg ggcgtttacg ggcgcaattt caccgtcgac 60
<210> 72
   <211> 61
   <212> DNA
   <213> Artificial Sequence
<220>
   <223> Description of Combined DNA/RNA Molecule: Primer
<220>
   <223> Description of Artificial Sequence: Primer
<400> 72
   aaataagaac cgaccacttu gccggagttc tgtttatacg gtttattctt ttccagcagc 60
   g 61
<210> 73
   <211> 69
   <212> DNA
   <213> Artificial Sequence
<220>
   <223> Description of Combined DNA/RNA Molecule: Primer
<220>
   <223> Description of Artificial Sequence: Primer
<400> 73
   aaagtggtcg gutcttattu cgtcgaggtg gcgcgcaact tccataagaa ggacgcggtg 60
   ctgcgtctg 69
<210> 74
   <211> 62
   <212> DNA
   <213> Artificial Sequence
<220>
   <223> Description of Combined DNA/RNA Molecule: Primer
<220>
   <223> Description of Artificial Sequence: Primer
<400> 74
   aaataagaac cgaccacttu cgcatcgctg gcgtccagcg tggcgatctt gccgctgccg 60
   tc 62
<210> 75
   <211> 63
   <212> DNA
   <213> Artificial Sequence
<220>
   <223> Description of Combined DNA/RNA Molecule: Primer
<220>
   <223> Description of Artificial Sequence: Primer
<400> 75
   aacctgaccc accugctgta cggctttauc ccgatctgcg gcggcaatgg catcaacgac 60
   agc                                                               63
<210> 76
   <211> 63
   <212> DNA
   <213> Artificial Sequence
<220>
   <223> Description of Combined DNA/RNA Molecule: Primer
<220>
   <223> Description of Artificial Sequence: Primer
<400> 76
   ataaagccgt acagcaggug ggtcaggtut tgcgccggga tcttgtcgac ggtgaaattg 60
   cgc                                                               63
<210> 77
   <211> 67
   <212> DNA
   <213> Artificial Sequence
<220>
   <223> Description of Combined DNA/RNA Molecule: Primer
<220>
   <223> Description of Artificial Sequence: Primer
<400> 77
   aacctgaccc accugctgta cggctttauc gatgacgaag gctggcgcat cgagatcccc 60
   ggcttgc                                                           67
<210> 78
   <211> 66
   <212> DNA
   <213> Artificial Sequence
<220>
   <223> Description of Combined DNA/RNA Molecule: Primer
<220>
   <223> Description of Artificial Sequence: Primer
<400> 78
   ataaagccgt acagcaggug ggtcaggtug taagccgcca tctgatccag cagacgcagc 60
   accgcg 66
<210> 79
   <211> 57
   <212> DNA
   <213> Artificial Sequence
<220>
   <223> Description of Combined DNA/RNA Molecule: Primer
<220>
   <223> Description of Artificial Sequence: Primer
<400> 79
   aaaatccugc cgtcgatcgg uggctggacg ctgtccgacc cgttcttctt catgggc 57
<210> 80
   <211> 57
   <212> DNA
   <213> Artificial Sequence
<220>
   <223> Description of Combined DNA/RNA Molecule: Primer
<220>
   <223> Description of Artificial Sequence: Primer
<400> 80
   accgatcgac ggcaggattt ucaggtcagg atgcgcctgc ttcagcgcca tcagctg 57
<210> 81
   <211> 62
   <212> DNA
   <213> Artificial Sequence
<220>
   <223> Description of Combined DNA/RNA Molecule: Primer
<220>
   <223> Description of Artificial Sequence: Primer
<400> 81
   aaaatccugc cgtcgatcgg uatgccggcg cacgcgcgcg ccgcggtggt ttcgatggaa 60
   gc 62
<210> 82
   <211> 63
   <212> DNA
   <213> Artificial Sequence
<220>
   <223> Description of Combined DNA/RNA Molecule: Primer
<220>
   <223> Description of Artificial Sequence: Primer
<400> 82
   accgatcgac ggcaggattt ucagctggcg cgcctgggcg tatttgatga tgtcgatgta 60
   gtc                                                               63
<210> 83
   <211> 70
   <212> DNA
   <213> Artificial Sequence
<220>
   <223> Description of Combined DNA/RNA Molecule: Primer
<220>
   <223> Description of Artificial Sequence: Primer
<400> 83
   atatcgactg ggagttcccg ggcgguaaag gcgccaaccc taacctgggc agcccgcaag 60
   acggggaaac                                                        70
<210> 84
   <211> 73
   <212> DNA
   <213> Artificial Sequence
<220>
   <223> Description of Combined DNA/RNA Molecule: Primer
<220>
   <223> Description of Artificial Sequence: Primer
<400> 84
   accgcccggg aactcccagt cgatauccac gccgaactct ttcaccgaac cgacgaagcg 60
   atcgcgcttc acc                                                    73
<210> 85
   <211> 68
   <212> DNA
   <213> Artificial Sequence
<220>
   <223> Description of Combined DNA/RNA Molecule: Primer
<220>
   <223> Description of Artificial Sequence: Primer
<400> 85
   atatcgactg ggagttcccg ggcgguaaac gcctgggcgc cggctatacc gacaaggcga 60
   aaccggaa                                                          68
<210> 86
   <211> 67
   <212> DNA
   <213> Artificial Sequence
<220>
   <223> Description of Combined DNA/RNA Molecule: Primer
<220>
   <223> Description of Artificial Sequence: Primer
<400> 86
   accgcccggg aactcccagt cgatauccac gcccttgatc ggctgcccgg cttctttatg 60
   catctgg 67
<210> 87
   <211> 53
   <212> DNA
   <213> Artificial Sequence
<220>
   <223> Description of Combined DNA/RNA Molecule: Primer
<220>
   <223> Description of Artificial Sequence: Primer
<400> 87
   agctgacctc cgccatcagu gccggtaagg acaagatcga caaggtggct tac 53
<210> 88
   <211> 55
   <212> DNA
   <213> Artificial Sequence
<220>
   <223> Description of Combined DNA/RNA Molecule: Primer
<220>
   <223> Description of Artificial Sequence: Primer
<400> 88
   actgatggcg gaggtcagcu catacttgcg gccggtttcc gccgacagct gatcc 55
<210> 89
   <211> 53
   <212> DNA
   <213> Artificial Sequence
<220>
   <223> Description of Combined DNA/RNA Molecule: Primer
<220>
   <223> Description of Artificial Sequence: Primer
<400> 89
   agctgacctc cgccatcagu ggcctgaaag acgccgaaag ttcgaaggcg ttc 53
<210> 90
   <211> 54
   <212> DNA
   <213> Artificial Sequence
<220>
   <223> Description of Combined DNA/RNA Molecule: Primer
<220>
   <223> Description of Artificial Sequence: Primer
<400> 90
   actgatggcg gaggtcagcu catcgatgcc gtgcgccttc accagcttgc tgac 54
<210> 91
   <211> 72
   <212> DNA
   <213> Artificial Sequence
<220>
   <223> Description of Combined DNA/RNA Molecule: Primer
<220>
   <223> Description of Artificial Sequence: Primer
<400> 91
   atggatcaca ucttcctgat gagutacgac ttctatggcg cgttcgatct gaagaacctg 60
   gggcatcaga cc 72
<210> 92
   <211> 70
   <212> DNA
   <213> Artificial Sequence
<220>
   <223> Description of Combined DNA/RNA Molecule: Primer
<220>
   <223> Description of Artificial Sequence: Primer
<400> 92
   aggagaacag gccgcccagc tgcttccacg gcttgtcgct tttggcgttg aagtggttgc 60
   cgtcgcggtc 70
<210> 93
   <211> 57
   <212> DNA
   <213> Artificial Sequence
<220>
   <223> Description of Combined DNA/RNA Molecule: Primer
<220>
   <223> Description of Artificial Sequence: Primer
<400> 93
   atggatcaca ucttcctgat gaguaccctg tattggggcg gtttcgacag cgttaac 57
<210> 94
   <211> 57
   <212> DNA
   <213> Artificial Sequence
<220>
   <223> Description of Combined DNA/RNA Molecule: Primer
<220>
   <223> Description of Artificial Sequence: Primer
<400> 94
   actcatcagg aagaugtgat ccaucgaggt ggcgaacgcc ttcgaacttt cggcgtc 57
<210> 95
   <211> 72
   <212> DNA
   <213> Artificial Sequence
<220>
   <223> Description of Combined DNA/RNA Molecule: Primer
<220>
   <223> Description of Artificial Sequence: Primer
<400> 95
   atcgtggucg gcaccgccat gtatggucgc ggctggaccg gggtgaacgg ctaccagaac 60
   aacattccgt tc 72
<210> 96
   <211> 72
   <212> DNA
   <213> Artificial Sequence
<220>
   <223> Description of Combined DNA/RNA Molecule: Primer
<220>
   <223> Description of Artificial Sequence: Primer
<400> 96
   atacatggcg gugccgacca cgatctugcc cggcttgacg ccctgcgcca gcagcgcatt 60
   gacgccgttc ac 72
<210> 97
   <211> 65
   <212> DNA
   <213> Artificial Sequence
<220>
   <223> Description of Combined DNA/RNA Molecule: Primer
<220>
   <223> Description of Artificial Sequence: Primer
<400> 97
   atcgtggucg gcaccgccat gtatggucct tacgaggtga atccggacga gcgcggttac 60
   tactg 65
<210> 98
   <211> 66
   <212> DNA
   <213> Artificial Sequence
<220>
   <223> Description of Combined DNA/RNA Molecule: Primer
<220>
   <223> Description of Artificial Sequence: Primer
<400> 98
   atacatggcg gugccgacca cgatctuata ccctttattg gcccagtcgt taacgctgtc 60
   gaaacc 66
<210> 99
   <211> 66
   <212> DNA
   <213> Artificial Sequence
<220>
   <223> Description of Combined DNA/RNA Molecule: Primer
<220>
   <223> Description of Artificial Sequence: Primer
<400> 99
   aagcagcugg gcggcctgtt ctccugggag atcgacgcgg ataacggcga tattctcaac 60
   agcatg 66
<210> 100
   <211> 60
   <212> DNA
   <213> Artificial Sequence
<220>
   <223> Description of Combined DNA/RNA Molecule: Primer
<220>
   <223> Description of Artificial Sequence: Primer
<400> 100
   aggagaacag gccgcccagc tgcttatcca gcacgtactt gcctttggcc tgcaccgagc 60
<210> 101
   <211> 68
   <212> DNA
   <213> Artificial Sequence
<220>
   <223> Description of Combined DNA/RNA Molecule: Primer
<220>
   <223> Description of Artificial Sequence: Primer
<400> 101
   aagcagcugg gcggcctgtt ctccugggag tggagcgaaa cccagcgcac cgatccgcag 60
   atggaata                                                          68
<210> 102
   <211> 70
   <212> DNA
   <213> Artificial Sequence
<220>
   <223> Description of Combined DNA/RNA Molecule: Primer
<220>
   <223> Description of Artificial Sequence: Primer
<400> 102
   aggagaacag gccgcccagc tgcttccacg gcttgtcgct tttggcgttg aagtggttgc 60
   cgtcgcggtc                                                        70
<210> 103
   <211> 455
   <212> PRT
   <213> Candida antarctica
<400> 103
   Met Arg Val Ser Leu Arg Ser Ile Thr Ser Leu Leu Ala Ala Ala Pro
    1               5                  10                  15
   Ala Ala Glu Thr Leu Asp Arg Arg Ala Ala Leu Pro Asn Pro Tyr Asp
               20                  25                  30
   Asp Pro Phe Tyr Thr Thr Pro Ser Asn Ile Gly Thr Phe Ala Lys Gly
           35                  40                  45
   Gln Val Ile Gln Ser Arg Lys Val Pro Thr Asp Ile Gly Asn Ala Asn
       50                  55                  60
   Asn Ala Ala Ser Phe Gln Leu Gln Tyr Arg Thr Thr Asn Thr Gln Asn
   65                  70                  75                  80
   Glu Ala Val Ala Asp Val Ala Thr Val Trp Ile Pro Ala Lys Pro Ala
                   85                  90                  95
   Ser Pro Pro Lys Ile Phe Ser Tyr Gln Val Tyr Glu Asp Ala Thr Ala
               100                 105                 110
   Leu Asp Cys Ala Pro Ser Tyr Ser Tyr Leu Thr Gly Leu Asp Gln Pro
           115                 120                 125
   Asn Lys Val Thr Ala Val Leu Asp Thr Pro Ile Ile Ile Gly Trp Ala
   130 135 140
   Leu Gln Gln Gly Tyr Tyr Val Val Ser Ser Asp His Glu Gly Phe Lys
   145 150 155 160
   Ala Ala Phe Ile Ala Gly Tyr Glu Glu Gly Met Ala Ile Leu Asp Gly
   165 170 175
   Ile Arg Ala Leu Lys Asn Tyr Gln Asn Leu Pro Ser Asp Ser Lys Val
   180 185 190
   Ala Leu Glu Gly Tyr Ser Gly Gly Ala His Ala Thr Val Trp Ala Thr
   195 200 205
   Ser Leu Ala Glu Ser Tyr Ala Pro Glu Leu Asn Ile Val Gly Ala Ser
   210 215 220
   His Gly Gly Thr Pro Val Ser Ala Lys Asp Thr Phe Thr Phe Leu Asn
   225 230 235 240
   Gly Gly Pro Phe Ala Gly Phe Ala Leu Ala Gly Val Ser Gly Leu Ser
   245 250 255
   Leu Ala His Pro Asp Met Glu Ser Phe Ile Glu Ala Arg Leu Asn Ala
   260 265 270
   Lys Gly Gln Arg Thr Leu Lys Gln Ile Arg Gly Arg Gly Phe Cys Leu
   275 280 285
   Pro Gln Val Val Leu Thr Tyr Pro Phe Leu Asn Val Phe Ser Leu Val
   290 295 300
   Asn Asp Thr Asn Leu Leu Asn Glu Ala Pro Ile Ala Ser Ile Leu Lys
   305 310 315 320
   Gln Glu Thr Val Val Gln Ala Glu Ala Ser Tyr Thr Val Ser Val Pro
   325 330 335
   Lys Phe Pro Arg Phe Ile Trp His Ala Ile Pro Asp Glu Ile Val Pro
   340 345 350
   Tyr Gln Pro Ala Ala Thr Tyr Val Lys Glu Gln Cys Ala Lys Gly Ala
   355 360 365
   Asn Ile Asn Phe Ser Pro Tyr Pro Ile Ala Glu His Leu Thr Ala Glu
   370 375 380
   Ile Phe Gly Leu Val Pro Ser Leu Trp Phe Ile Lys Gln Ala Phe Asp
   385 390 395 400
   Gly Thr Thr Pro Lys Val Ile Cys Gly Thr Pro Ile Pro Ala Ile Ala
   405 410 415
   Gly Ile Thr Thr Pro Ser Ala Asp Gln Val Leu Gly Ser Asp Leu Ala
   420 425 430
   Asn Gln Leu Arg Ser Leu Asp Gly Lys Gln Ser Ala Phe Gly Lys Pro
   435 440 445
   Phe Gly Pro Ile Thr Pro Pro
   450 455
<210> 104
   <211> 468
   <212> PRT
   <213> Candida albicans
<400> 104
   Met Arg Gly Ile Ala Val Phe Leu Ala Phe Ile Ser Leu Ile Phe Ala
   1 5 10 15
   Ser Pro Leu Thr Val Lys Ser Pro Leu Val Asp Asp Phe Tyr Thr Ala
   20 25 30
   Pro Asp Gly Tyr Glu Ser Ala Lys Leu Gly Glu Ile Leu Lys Leu Arg
   35 40 45
   Lys Thr Pro Ser Lys Leu Ser Ser Met Phe Phe Glu Ile Asp Ile Lys
   50 55 60
   Asn Ser Trp Gln Leu Leu Val Arg Ser Glu Asp Ser Phe Gly Asn Ala
   65 70 75 80
   Thr Ala Ile Val Thr Thr Val Ile Glu Pro Tyr Asn Ala Asp Pro Ser
   85 90 95
   Lys Val Leu Ser Tyr Gln Thr Phe Glu Asp Ser Ala Asn Ile Glu Cys
   100 105 110
   Ser Pro Ser Tyr Gly Met Gln Tyr Gly Ala Pro Trp Ser Thr Val Ala
   115 120 125
   Thr Gln Ile Asp Met Ala Leu Met Val Pro Met Leu Lys Gln Gly Tyr
   130 135 140
   Tyr Val Val Ser Pro Asp Tyr Glu Gly Pro Lys Ser Thr Phe Thr Val
   145 150 155 160
   Gly Arg Gln Ser Gly Lys Ala Thr Leu Asp Ser Ile Arg Ala Ile Leu
   165 170 175
   Lys Ser Asn Lys Phe Thr Gly Ile Lys Ser Asp Ala Lys Val Ala Met
   180 185 190
   Trp Gly Tyr Ser Gly Gly Ser Leu Ala Ser Gly Trp Ala Ala Ala Leu
   195 200 205
   Gln Pro Lys Tyr Ala Pro Glu Leu Lys Lys Asn Leu Ile Gly Ala Ala
   210 215 220
   Leu Gly Gly Phe Val Thr Asn Ile Thr Ala Thr Ala Glu Ala Thr Asp
   225 230 235 240
   Gly Thr Leu Phe Ala Gly Leu Val Pro Asn Ala Leu Ser Gly Leu Ala
   245 250 255
   Asn Glu Tyr Pro Glu Phe Lys Glu Ile Leu Tyr Gln Lys Val Ser Lys
   260 265 270
   Ala Ala Thr Asp Asn Leu Arg Gln Gly Thr Glu His Cys Ile Gly Gly
   275 280 285
   Ala Ile Leu Tyr Phe Ala Glu Asp Gln Tyr Phe Thr Gly Asp Asp Arg
   290 295 300
   Ala Phe Pro Gly Gly Tyr Gly Leu Leu Lys Glu Glu Val Val Asn Lys
   305 310 315 320
   Thr Ile Ser Glu Asn Asn Leu Met Gln Met Asp Lys Asp Tyr Leu Pro
   325 330 335
   Asp Ile Pro Ile Phe Val Tyr His Gly Ala Leu Asp Ser Ile Val Pro
   340 345 350
   Ile Ser Asn Val His Val Thr Tyr Lys Asn Trp Cys Asp Trp Gly Ile
   355 360 365
   Asn Ser Phe Glu Phe Ser Glu Asp Leu Leu Asn Gly His Ile Thr Glu
   370 375 380
   Thr Ile Val Gly Ala Pro Ala Ala Ile Thr Trp Leu Glu Ala Arg Phe
   385 390 395 400
   Asp Gly Glu Pro Val Val Lys Gly Cys Lys Lys Thr Ser Arg Ile Thr
   405 410 415
   Asn Phe Ser Tyr Pro Asn Ile Ser Asp Ser Thr Ser Ser Ile Phe Glu
   420 425 430
   Gly Ile Leu Asn Ser Val Thr Gly Ser Glu Leu Gly Pro Gly Val Thr
   435 440 445
   Ser Asp Asn Ile Thr Leu Asp Gly Leu Thr Gly Phe Leu Gly Asn Phe
   450 455 460
   Ile Asp Leu Lys
   465
<210> 105
   <211> 459
   <212> PRT
   <213> Candida albicans
<400> 105
   Met Leu Phe Leu Leu Phe Leu Leu Val Ala Pro Ile Tyr Ala Gly Leu
   1 5 10 15
   Ile Leu Pro Thr Lys Pro Ser Asn Asp Pro Phe Tyr Asn Ala Pro Ala
               20                  25                  30
   Gly Phe Glu Lys Ala Ala Val Gly Asp Ile Leu Gln Ser Arg Lys Thr
           35                  40                  45
   Pro Lys Pro Ile Thr Gly Val Phe Val Pro Val Lys Ile Gln Asn Ser
       50                  55                  60
   Trp Gln Leu Leu Val Arg Ser Glu Asp Ser Phe Gly Asn Pro Asn Val
   65                  70                  75                  80
   Ile Val Thr Thr Val Met Glu Pro Phe Asn Ala Asp Pro Ser Lys Leu
                   85                  90                  95
   Ala Ser Tyr Gln Val Phe Glu Asp Ser Ala Lys Ala Asp Cys Ala Pro
               100                 105                 110
   Ser Tyr Ala Leu Gln Phe Gly Ser Asp Val Thr Thr Ile Ala Thr Gln
           115                 120                 125
   Val Glu Thr Tyr Leu Leu Ala Pro Leu Leu Asp Gln Gly Tyr Tyr Val
       130                 135                 140
   Val Ser Pro Asp Tyr Glu Gly Pro Lys Ser Thr Phe Thr Val Gly Lys
   145                 150                 155                 160
   Gln Ser Gly Gln Ala Val Leu Asn Ser Ile Arg Ala Ala Leu Lys Ser
                   165                 170                 175
   Gly Lys Ile Thr Asn Leu Ala Glu Asn Ala Lys Val Val Met Trp Gly
               180                 185                 190
   Tyr Ser Gly Gly Ser Leu Ala Ser Gly Trp Ala Ala Ala Leu Gln Pro
           195                 200                 205
   Asn Tyr Ala Pro Glu Leu Gly Gly Asn Leu Leu Gly Ala Ala Leu Gly
       210                 215                 220
   Gly Phe Val Thr Asn Ile Thr Ala Thr Ala Glu Ala Thr Asp Gly Thr
   225                 230                 235                 240
   Val Phe Ala Gly Ile Met Ala Asn Ala Leu Gly Gly Val Ala Asn Glu
                   245                 250                 255
   Tyr Pro Glu Phe Lys Gln Ile Leu Gln Asn Asp Thr Asp Lys Gln Ser
               260                 265                 270
   Val Phe Asp Gln Phe Asp Asn His Cys Leu Ala Asp Gly Val Ile Asn
           275                 280                 285
   Tyr Ile Gly Lys His Phe Leu Ser Gly Thr Asn Lys Ile Phe Lys Ser
       290                 295                 300
   Gly Trp Asn Ile Leu Lys Asn Pro Thr Ile Ser Lys Ile Val Glu Asp
   305                 310                 315                 320
   Asn Gly Leu Val Tyr Gln Lys Gln Leu Val Pro Lys Ile Pro Ile Leu
   325 330 335
   Ile Tyr His Gly Ala Ile Asp Gln Ile Val Pro Ile Val Asn Val Lys
   340 345 350
   Lys Thr Tyr Gln Asn Trp Cys Asp Ala Gly Ile Ala Ser Leu Glu Phe
   355 360 365
   Ser Glu Asp Ala Thr Asn Gly His Ile Thr Glu Thr Ile Val Gly Ala
   370 375 380
   Pro Val Ala Leu Thr Trp Ile Ile Asn Arg Phe Asn Gly Lys Gln Thr
   385 390 395 400
   Val Ser Gly Cys Gln His Val Lys Arg Thr Ser Asn Phe Glu Tyr Pro
   405 410 415
   Asn Ile Pro Pro Ser Ile Leu Asn Tyr Phe Lys Ala Ala Leu Asn Ile
   420 425 430
   Leu Ile Gln Lys Gly Leu Gly Pro Asp Ile Gln Lys Asp Gln Val Asn
   435 440 445
   Pro Asp Gly Leu Lys Lys Ile Ser Ile Leu Val
   450 455
<210> 106
   <211> 465
   <212> PRT
   <213> Candida parapsilosis
<400> 106
   Met His Phe Trp Phe Leu Ser Ile Phe Leu Ile Gln Gly Val Phe Ala
   1 5 10 15
   Ala Val Ile Ala Pro Val Lys Pro Ser Gln Asp Asn Phe Tyr Thr Pro
   20 25 30
   Pro Asp Gly Tyr Glu Asn Ser Lys Val Gly Thr Ile Leu Lys Phe Arg
   35 40 45
   Asn Thr Pro Phe Pro Leu Ser Gly Ile Ile Asn Thr Val Asn Val Gln
   50 55 60
   Asn Ser Trp Gln Leu Leu Val Arg Ser Glu Asp Thr Phe Gly Asn Pro
   65 70 75 80
   Asn Ala Ile Val Thr Thr Val Ile Gln Pro Phe Asn Ala Thr Ser Asp
   85 90 95
   Lys Val Val Ser Tyr Gln Thr Trp Glu Asp Ala Ala Asn Leu Asp Phe
   100 105 110
   Ser Pro Ser Tyr Gly Ile Gln Tyr Gly Ala Asp Leu Thr Thr Leu Ile
           115                 120                 125
   Ser Ser Phe Glu Met Tyr Phe Met Ser Ala Leu Leu Asp Gln Gly Tyr
       130                 135                 140
   Tyr Val Val Thr Pro Asp Tyr Glu Gly Pro Lys Ser Thr Phe Thr Val
   145                 150                 155                 160
   Gly Leu Gln Ser Gly Lys Ala Thr Leu Asn Ser Ile Arg Ala Ala Leu
                   165                 170                 175
   Gly Ser Gly Asn Leu Thr Gly Ile Asp Ser Asn Ala Glu Val Met Met
               180                 185                 190
   Trp Gly Tyr Ser Gly Gly Thr Ile Ala Ser Gly Trp Ala Ala Ala Ile
           195                 200                 205
   Gln Ser Glu Tyr Ala Pro Glu Leu Thr Asp Asn Leu Ile Gly Ala Ala
       210                 215                 220
   Leu Gly Gly Phe Val Thr Asn Ile Thr Ala Thr Ala Glu Ala Thr Asp
   225                 230                 235                 240
   Gly Gly Leu Phe Ala Gly Leu Ile Ala Asn Ala Leu Gly Gly Leu Gly
                   245                 250                 255
   Asn Glu Tyr Ser Asn Leu Thr Thr Phe Met Gln Glu Ser Val Ser Ser
               260                 265                 270
   Glu Gln Gln Pro Ala Phe Glu Arg Arg Asp Asp His Cys Leu Val Asp
           275                 280                 285
   Ser Ile Leu Gly Asn Ile Gly Ser Gln Phe Phe Ser Gly Glu Asp Arg
       290                 295                 300
   Trp Phe Pro Asp Gly Trp Lys Ile Phe Asp Glu Glu Pro Val Asp Ser
   305                 310                 315                 320
   Ile Val Lys Asn Asn Gly Leu Val Tyr Gln Pro Lys Lys Tyr Leu Pro
                   325                 330                 335
   Gln Ile Pro Ile Phe Val Tyr Gln Gly Thr Gln Asp Asn Ile Val Pro
               340                 345                 350
   Ile Lys Ser Ala Lys Thr Thr Phe Lys Gln Trp Cys Glu Trp Gly Leu
           355                 360                 365
   Glu Ser Gly Glu Phe Ala Glu Asp Glu Ala Thr Gly His Ile Thr Glu
       370                 375                 380
   Val Phe Val Gly Ala Pro Ala Ala Leu Thr Trp Ile Ile Asn Arg Phe
   385                 390                 395                 400
   Asn Gly Val Glu Pro Val Gln Gly Cys Asn His Thr Ser Arg Ala Ser
                   405                 410                 415
   Asn Phe Asp Tyr Pro Gly Ile Ser Gln Ser Tyr Val Glu Tyr Phe Thr
   420 425 430
   Ala Ala Leu Asn Val Val Leu Gly Ile Asn Met Gly Pro Leu Thr Lys
   435 440 445
   Arg Glu Val Asn Ser Ile Gln Asp Leu Asn Asn Leu Glu Tyr Val Lys
   450 455 460
   Val
   465
<210> 107
   <211> 7
   <212> PRT
   <213> Artificial Sequence
<220>
   <223> Description of Artificial Sequence: Consesus sequence peptide fragment
<400> 107
   Ser Tyr Gln Val Tyr Glu Asp
   1 5
<210> 108
   <211> 7
   <212> PRT
   <213> Artificial Sequence
<220>
   <223> Description of Artificial Sequence: Consesus sequence peptide fragment
<400> 108
   Gln Gly Tyr Tyr Val Val Ser
   1 5
<210> 109
   <211> 7
   <212> PRT
   <213> Artificial Sequence
<220>
   <223> Description of Artificial Sequence: Consesus sequence peptide fragment
<400> 109
   Val Gly Ala Ser His Gly Gly
   1 5
<210> 110
   <211> 9
   <212> PRT
   <213> Artificial Sequence
<220>
   <223> Description of Artificial Sequence: Consesus sequence peptide fragment
<400> 110
   Pro Lys Phe Pro Arg Phe Ile Trp His
    1               5
<210> 111
   <211> 8
   <212> PRT
   <213> Artificial Sequence
<220>
   <223> Description of Artificial Sequence: Consesus sequence peptide fragment
<400> 111
   Leu Trp Phe Ile Lys Gln Ala Phe
    1               5
<210> 112
   <211> 21
   <212> DNA
   <213> Artificial Sequence
<220>
   <223> Description of Artificial Sequence: Synthetic modified nucleotide sequence
<400> 112
   agttaccagg tctacgagga t 21
<210> 113
   <211> 21
   <212> DNA
   <213> Artificial Sequence
<220>
   <223> Description of Artificial Sequence: Synthetic modified nucleotide sequence
<400> 113
   cagggctact acgtcgtctc t 21
<210> 114
   <211> 21
   <212> DNA
   <213> Artificial Sequence
<220>
   <223> Description of Artificial Sequence: Synthetic modified nucleotide sequence
<400> 114
   gtaggtgctt cgcacggagg t 21
<210> 115
   <211> 27
   <212> DNA
   <213> Artificial Sequence
<220>
   <223> Description of Artificial Sequence: Synthetic modified nucleotide sequence
<400> 115
   cccaagttcc cgcgcttcat ctggcat 27
<210> 116
   <211> 24
   <212> DNA
   <213> Artificial Sequence
<220>
   <223> Description of Artificial Sequence: Synthetic modified nucleotide sequence
<400> 116
   ttatggttta tcaagcaagc gttc 24
<210> 117
   <211> 854
   <212> PRT
   <213> Serratia marcescens
<400> 117
   Asp Gln Gln Leu Val Asp Gln Leu Ser Gln Leu Lys Leu Asn Val Lys
   1               5                   10                  15
   Met Leu Asp Asn Arg Ala Gly Glu Asn Gly Val Asp Cys Ala Ala Leu
               20                  25                  30
   Gly Ala Asp Trp Ala Ser Cys Asn Arg Val Leu Phe Thr Leu Ser Asn
           35                  40                  45
   Asp Gly Gln Ala Ile Asp Gly Lys Asp Trp Val Ile Tyr Phe His Ser
       50                  55                  60
   Pro Arg Gln Thr Leu Arg Val Asp Asn Asp Gln Phe Lys Ile Ala His
   65                  70                  75                  80
   Leu Thr Gly Asp Leu Tyr Lys Leu Glu Pro Thr Ala Lys Phe Ser Gly
                   85                  90                  95
   Phe Pro Ala Gly Lys Ala Val Glu Ile Pro Val Val Ala Glu Tyr Trp
   100 105 110
   Gln Leu Phe Arg Asn Asp Phe Leu Pro Arg Trp Tyr Ala Thr Ser Gly
   115 120 125
   Asp Ala Lys Pro Lys Met Leu Ala Asn Thr Asp Thr Glu Asn Leu Asp
   130 135 140
   Gln Phe Val Ala Pro Phe Thr Gly Asp Gln Trp Lys Arg Thr Lys Asp
   145 150 155 160
   Asp Lys Asn Ile Leu Met Thr Pro Ala Ser Arg Phe Val Ser Asn Ala
   165 170 175
   Asp Leu Gln Thr Leu Pro Ala Gly Ala Leu Arg Gly Lys Ile Val Pro
   180 185 190
   Thr Pro Met Gln Val Lys Val His Ala Gln Asp Ala Asp Leu Arg Lys
   195 200 205
   Gly Val Ala Leu Asp Leu Ser Thr Leu Val Lys Pro Ala Ala Asp Val
   210 215 220
   Val Ser Gln Arg Phe Ala Leu Leu Gly Val Pro Val Gln Thr Asn Gly
   225 230 235 240
   Tyr Pro Ile Lys Thr Asp Ile Gln Pro Gly Lys Phe Lys Gly Ala Met
   245 250 255
   Ala Val Ser Gly Ala Tyr Glu Leu Lys Ile Gly Lys Lys Glu Ala Gln
   260 265 270
   Val Ile Gly Phe Asp Gln Ala Gly Val Phe Tyr Gly Leu Gln Ser Ile
   275 280 285
   Leu Ser Leu Val Pro Ser Asp Gly Ser Gly Lys Ile Ala Thr Leu Asp
   290 295 300
   Ala Ser Asp Ala Pro Arg Phe Pro Tyr Arg Gly Ile Phe Leu Asp Val
   305 310 315 320
   Ala Arg Asn Phe His Lys Lys Asp Ala Val Leu Arg Leu Leu Asp Gln
   325 330 335
   Met Ala Ala Tyr Lys Leu Asn Lys Phe His Phe His Leu Ser Asp Asp
   340 345 350
   Glu Gly Trp Arg Ile Glu Ile Pro Gly Leu Pro Glu Leu Thr Glu Val
   355 360 365
   Gly Gly Gln Arg Cys His Asp Leu Ser Glu Thr Thr Cys Leu Leu Pro
   370 375 380
   Gln Tyr Gly Gln Gly Pro Asp Val Tyr Gly Gly Phe Phe Ser Arg Gln
   385 390 395 400
   Asp Tyr Ile Asp Ile Ile Lys Tyr Ala Gln Ala Arg Gln Ile Glu Val
   405 410 415
   Ile Pro Glu Ile Asp Met Pro Ala His Ala Arg Ala Ala Val Val Ser
   420 425 430
   Met Glu Ala Arg Tyr Lys Lys Leu His Ala Ala Gly Lys Glu Gln Glu
   435 440 445
   Ala Asn Glu Phe Arg Leu Val Asp Pro Thr Asp Thr Ser Asn Thr Thr
   450 455 460
   Ser Val Gln Phe Phe Asn Arg Gln Ser Tyr Leu Asn Pro Cys Leu Asp
   465 470 475 480
   Ser Ser Gln Arg Phe Val Asp Lys Val Ile Gly Glu Ile Ala Gln Met
   485 490 495
   His Lys Glu Ala Gly Gln Pro Ile Lys Thr Trp His Phe Gly Gly Asp
   500 505 510
   Glu Ala Lys Asn Ile Arg Leu Gly Ala Gly Tyr Thr Asp Lys Ala Lys
   515 520 525
   Pro Glu Pro Gly Lys Gly Ile Ile Asp Gln Gly Asn Glu Asp Lys Pro
   530 535 540
   Trp Ala Lys Ser Gln Val Cys Gln Thr Met Ile Lys Glu Gly Lys Val
   545 550 555 560
   Ala Asp Met Glu His Leu Pro Ser Tyr Phe Gly Gln Glu Val Ser Lys
   565 570 575
   Leu Val Lys Ala His Gly Ile Asp Arg Met Gln Ala Trp Gln Asp Gly
   580 585 590
   Leu Lys Asp Ala Glu Ser Ser Lys Ala Phe Ala Thr Ser Arg Val Gly
   595 600 605
   Val Asn Phe Trp Asp Thr Leu Tyr Trp Gly Gly Phe Asp Ser Val Asn
   610 615 620
   Asp Trp Ala Asn Lys Gly Tyr Glu Val Val Val Ser Asn Pro Asp Tyr
   625 630 635 640
   Val Tyr Met Asp Phe Pro Tyr Glu Val Asn Pro Asp Glu Arg Gly Tyr
   645 650 655
   Tyr Trp Gly Thr Arg Phe Ser Asp Glu Arg Lys Val Phe Ser Phe Ala
   660 665 670
   Pro Asp Asn Met Pro Gln Asn Ala Glu Thr Ser Val Asp Arg Asp Gly
   675 680 685
   Asn His Phe Asn Ala Lys Ser Asp Lys Pro Trp Pro Gly Ala Tyr Gly
   690 695 700
   Leu Ser Ala Gln Leu Trp Ser Glu Thr Gln Arg Thr Asp Pro Gln Met
   705 710 715 720
   Glu Tyr Met Ile Phe Pro Arg Ala Leu Ser Val Ala Glu Arg Ala Gly
   725 730 735
   Trp Glu Gln Asp Tyr Arg Ala Gly Arg Glu Tyr Lys Gly Gly Glu Thr
   740 745 750
   His Phe Val Asp Thr Gln Ala Leu Glu Lys Asp Trp Leu Arg Phe Ala
   755 760 765
   Asn Ile Leu Gly Gln Arg Glu Leu Ala Lys Leu Asp Lys Gly Gly Val
   770 775 780
   Ala Tyr Arg Leu Pro Val Pro Gly Ala Arg Val Ala Gly Gly Lys Leu
   785 790 795 800
   Glu Ala Asn Ile Ala Leu Pro Gly Leu Gly Ile Glu Tyr Ser Thr Asp
   805 810 815
   Gly Gly Lys Gln Trp Gln Arg Tyr Asp Ala Lys Ala Lys Pro Ala Val
   820 825 830
   Ser Gly Glu Val Gln Val Arg Ser Val Ser Pro Asp Gly Lys Arg Tyr
   835 840 845
   Ser Arg Ala Glu Lys Val
   850
<210> 118
   <211> 540
   <212> PRT
   <213> Serratia marcescens
<400> 118
   Ala Ala Pro Gly Lys Pro Thr Ile Ala Trp Gly Asn Thr Lys Phe Ala
   1 5 10 15
   Ile Val Glu Val Asp Gln Ala Ala Thr Ala Tyr Asn Asn Leu Val Lys
   20 25 30
   Val Lys Asn Ala Ala Asp Val Ser Val Ser Trp Asn Leu Trp Asn Gly
   35 40 45
   Asp Thr Gly Thr Thr Ala Lys Val Leu Leu Asn Gly Lys Glu Ala Trp
   50 55 60
   Ser Gly Pro Ser Thr Gly Ser Ser Gly Thr Ala Asn Phe Lys Val Asn
   65 70 75 80
   Lys Gly Gly Arg Tyr Gln Met Gln Val Ala Leu Cys Asn Ala Asp Gly
   85 90 95
   Cys Thr Ala Ser Asp Ala Thr Glu Ile Val Val Ala Asp Thr Asp Gly
   100 105 110
   Ser His Leu Ala Pro Leu Lys Glu Pro Leu Leu Glu Lys Asn Lys Pro
   115 120 125
   Tyr Lys Gln Asn Ser Gly Lys Val Val Gly Ser Tyr Phe Val Glu Trp
   130 135 140
   Gly Val Tyr Gly Arg Asn Phe Thr Val Asp Lys Ile Pro Ala Gln Asn
   145 150 155 160
   Leu Thr His Leu Leu Tyr Gly Phe Ile Pro Ile Cys Gly Gly Asn Gly
   165 170 175
   Ile Asn Asp Ser Leu Lys Glu Ile Glu Gly Ser Phe Gln Ala Leu Gln
   180 185 190
   Arg Ser Cys Gln Gly Arg Glu Asp Phe Lys Val Ser Ile His Asp Pro
   195 200 205
   Phe Ala Ala Leu Gln Lys Ala Gln Lys Gly Val Thr Ala Trp Asp Asp
   210 215 220
   Pro Tyr Lys Gly Asn Phe Gly Gln Leu Met Ala Leu Lys Gln Ala His
   225 230 235 240
   Pro Asp Leu Lys Ile Leu Pro Ser Ile Gly Gly Trp Thr Leu Ser Asp
   245 250 255
   Pro Phe Phe Phe Met Gly Asp Lys Val Lys Arg Asp Arg Phe Val Gly
   260 265 270
   Ser Val Lys Glu Phe Leu Gln Thr Trp Lys Phe Phe Asp Gly Val Asp
   275 280 285
   Ile Asp Trp Glu Phe Pro Gly Gly Lys Gly Ala Asn Pro Asn Leu Gly
   290 295 300
   Ser Pro Gln Asp Gly Glu Thr Tyr Val Leu Leu Met Lys Glu Leu Arg
   305 310 315 320
   Ala Met Leu Asp Gln Leu Ser Val Glu Thr Gly Arg Lys Tyr Glu Leu
   325 330 335
   Thr Ser Ala Ile Ser Ala Gly Lys Asp Lys Ile Asp Lys Val Ala Tyr
   340 345 350
   Asn Val Ala Gln Asn Ser Met Asp His Ile Phe Leu Met Ser Tyr Asp
   355 360 365
   Phe Tyr Gly Ala Phe Asp Leu Lys Asn Leu Gly His Gln Thr Ala Leu
   370 375 380
   Asn Ala Pro Ala Trp Lys Pro Asp Thr Ala Tyr Thr Thr Val Asn Gly
   385 390 395 400
   Val Asn Ala Leu Leu Ala Gln Gly Val Lys Pro Gly Lys Ile Val Val
                   405                 410                 415
   Gly Thr Ala Met Tyr Gly Arg Gly Trp Thr Gly Val Asn Gly Tyr Gln
               420                 425                 430
   Asn Asn Ile Pro Phe Thr Gly Thr Ala Thr Gly Pro Val Lys Gly Thr
           435                 440                 445
   Trp Glu Asn Gly Ile Val Asp Tyr Arg Gln Ile Ala Gly Gln Phe Met
       450                 455                 460
   Ser Gly Glu Trp Gln Tyr Thr Tyr Asp Ala Thr Ala Glu Ala Pro Tyr
   465                 470                 475                 480
   Val Phe Lys Pro Ser Thr Gly Asp Leu Ile Thr Phe Asp Asp Ala Arg
                   485                 490                 495
   Ser Val Gln Ala Lys Gly Lys Tyr Val Leu Asp Lys Gln Leu Gly Gly
               500                 505                 510
   Leu Phe Ser Trp Glu Ile Asp Ala Asp Asn Gly Asp Ile Leu Asn Ser
           515                 520                 525
   Met Asn Ala Ser Leu Gly Asn Ser Ala Gly Val Gln
       530                 535                 540
<210> 119
   <211> 12
   <212> PRT
   <213> Artificial Sequence
<220>
   <223> Description of Artificial Sequence: Consensus sequence peptide fragment
<400> 119
   Asn Ser Gly Lys Val Val Gly Ser Tyr Phe Val Glu
   1               5                   10
<210> 120
   <211> 10
   <212> PRT
   <213> Artificial Sequence
<220>
   <223> Description of Artificial Sequence: Consensus sequence peptide fragment
<400> 120
   Asn Leu Thr His Leu Leu Tyr Gly Phe Ile
   1               5                   10
<210> 121
   <211> 8
   <212> PRT
   <213> Artificial Sequence
<220>
   <223> Description of Artificial Sequence: Consensus sequence peptide fragment
<400> 121
   Leu Lys Ile Leu Pro Ser Ile Gly
   1 5
<210> 122
   <211> 12
   <212> PRT
   <213> Artificial Sequence
<220>
   <223> Description of Artificial Sequence: Consensus sequence peptide fragment
<400> 122
   Gly Val Asp Ile Asp Trp Glu Phe Pro Gly Gly Lys
   1 5 10
<210> 123
   <211> 7
   <212> PRT
   <213> Artificial Sequence
<220>
   <223> Description of Artificial Sequence: Consensus sequence peptide fragment
<400> 123
   Glu Leu Thr Ser Ala Ile Ser
   1 5
<210> 124
   <211> 8
   <212> PRT
   <213> Artificial Sequence
<220>
   <223> Description of Artificial Sequence: Consensus sequence peptide fragment
<400> 124
   Met Asp His Ile Phe Leu Met Ser
   1 5
<210> 125
   <211> 5
   <212> PRT
   <213> Artificial Sequence
<220>
   <223> Description of Artificial Sequence: Consensus sequence peptide fragment
<400> 125
   Lys Ile Val Val Gly
   1 5
<210> 126
   <211> 5
   <212> PRT
   <213> Artificial Sequence
<220>
   <223> Description of Artificial Sequence: Consensus sequence peptide fragment
<400> 126
   Thr Ala Met Tyr Gly
   1 5
<210> 127
   <211> 10
   <212> PRT
   <213> Artificial Sequence
<220>
   <223> Description of Artificial Sequence: Consensus sequence peptide fragment
<400> 127
   Lys Gln Leu Gly Gly Leu Phe Ser Trp Glu
   1 5 10

## Description

### FIELD OF THE INVENTION

The present invention relates to libraries comprising recombinant chimeric proteins, each protein comprising a plurality of distinct consensus amino acid sequences corresponding to a structure or an amino acid sequence that are conserved in a plurality of functionally and/or structurally related proteins. The 10 present invention further relates to methods for preparing the recombinant chimeric proteins and uses thereof that are less expensive, less work-intensive and more efficient than procedures used in current available methods. The advantage of the present invention is that shuffling between variable regions that are not necessarily predetermined, while maintaining the consensus backbone, 15 increases the production of active enzymes while keeping high diversity, thereby, more favorable and important enzyme variants are generated.

### BACKGROUND OF THE INVENTION

For certain industrial and pharmacological needs, it is required to modify and further to improve the characteristics of native proteins. Improvement can be20 achieved by introducing single or multiple mutations into the genes encoding the desired proteins, in a process that is commonly termed 'directed evolution'. This process involves repeated cycles of random mutagenesis following product selection until the desired result is achieved.

Single point mutations have relatively low improvement potential, and thus25 strategies for screening products carrying preferably multiple mutations, such as, error-prone polymerase chain reaction and cassette mutagenesis where the specific region to be optimized is replaced with a synthetically mutagenized oligonucleotide. The latter approach is preferred for the construction of protein libraries. Error-prone PCR uses low-fidelity polymerization conditions to30 introduce a considerable level of point mutations randomly over a long sequence. Some computer simulations have suggested that point mutagenesis alone may often be too gradual to allow the large-scale block changes that are required for continued and dramatic sequence evolution. In addition, repeated cycles of error-prone PCR can lead to an accumulation of neutral mutations with undesired results, such as affecting a protein's immunogenicity but not its binding affinity. Above all, a serious limitation of error-prone PCR is that the rate of negative mutations grows with the sensitivity of the mutated regions to random mutagenesis. This sensitivity is also referred as information density'.

Information density is the information content per unit length of a sequence, wherein 'information content' or IC, is defined as the resistance of the active protein to the amino acid sequence variation. IC is calculated from the minimum number of invariable amino acids required to describe a family of functionally-related sequences. This parameter is used to classify the complexity of an active sequence of a biological macromolecule (e.g., polynucleotide or polypeptide). Thus, regions in proteins that are relatively sensitive to random mutagenesis, are considered as having a high information density and are often found conserved throughout evolution.

In cassette mutagenesis, a sequence block in a single template is replaced by a sequence that was fully, or partially, randomized. Accordingly, the number of random sequences applied limits the maximum IC that may be obtained, further eliminating potential sequences from being included in the libraries. This procedure also requires sequencing of individual clones after each selection round, which is tedious and impractical for many rounds of mutagenesis. Error-prone PCR and cassette mutagenesis are therefore widely used for fine-tuning of comparatively low IC.

Evolution of most organisms occurs by natural selection and sexual reproduction, which ensures the mixing and combining of the genes in the offspring of the selected individuals. During meiosis, homologous chromosomes from the parents line up with one another and by crossing-over parts along their sequences, namely via recombination, are randomly swapping genetic material. In many events, since the introduced sequences had a proven utility prior to recombination, they maintain a substantial IC in the new environment.

DNA shuffling is a process directed at accelerating the improvement potential of directed evolution by generating extensive recombinations in vitro and in vivo between mutants possessing improved traits. The outlines of this process include: induction of random or cassette mutagenesis, selection, cleaving mutant genes of choice into segments by a variety of methods and inducing recombination between the various segments by a variety of methods.

US Patent No. 6,573,098 discloses compositions comprising a library of nucleic acids comprising a composition of a plurality of overlapping nucleic acids, which are segments of the same gene from different species, are capable of hybridizing to a portion of a selected target nucleic acid or set of related sequence target nucleic acids, comprise one or more region of non-complementarity with the selected target nucleic acid, are capable of priming nucleotide extension upon hybridization to the selected target nucleic acid, and wherein the selected target nucleic acid is one of the genes used to provide the plurality of overlapping nucleic acids. In a preferred embodiment of US 6,573,098 the plurality of overlapping nucleic acids used for DNA shuffling comprise regions of at least 50 consecutive nucleotides which have at least percent sequence identity, preferably at least 90 percent sequence identity.

US Patent No. 6,489,145 discloses a method for producing hybrid polynucleotides comprising: creating mutations in samples of nucleic acid sequences; optionally screening for desired characteristics within the mutagenized samples; and transforming a plurality of host cells with nucleic acid sequences having said desired characteristics, wherein said one or more nucleic acid sequences include at least a first polynucleotide that shares at least one region of partial sequence homology with a second polynucleotide in the host cell; wherein said partial sequence homology promotes reassortment processes which result in sequence reorganization; thereby producing said hybrid polynucleotides. This method is conducted in vivo, utilizing cellular processes to form the hybrid polynucleotides.

DNA family shuffling is a modified DNA shuffling process, which introduces evolutionary changes that are more significant than point mutations while maintaining sequence coherency. This process involves usage of a parental DNA as a template for the same gene from different organisms.

US Patent Nos. 6,479,652 discloses compositions and methods for family shuffling procedure. In these methods, sets of overlapping family gene shuffling oligonucleotides are hybridized and elongated, providing a population of recombined nucleic acids, which can be selected for a desired trait or property. Typically, the set of overlapping family shuffling gene oligonucleotides include a plurality of oligonucleotide member types derived from a plurality of homologous target nucleic acids.

WO 01/75091 A discloses a method for producing a polynucleotide sequence encoding an antibody variable domain, the variable domain comprising complementarity-determining regions (CDRs) located within a selected framework

US Patent Nos. 6,479,652 discloses compositions and methods for family shuffling procedure. In these methods, sets of overlapping family gene shuffling oligonucleotides are hybridized and elongated, providing a population of recombined nucleic acids, which can be selected for a desired trait or property.

WO 03/044198 A discloses an artificial antibody library that is constructed by amplifying a fragment of the VH or VL region of an immunoglobulin gene" integrating the VH library and the VL library into an non-expression vector, transferring it into a host and then shuffling the VH region in the VH library with the VL region in the VL library. Particularly it relates to variability and modification of protein function by shuffling polymerisation shuffling sequence segments.

Soderlind E et al discloses constructing a single-chain Fv antibody library that permits human complementarity-determining region (CDR) gene fragments of any germline to be incorporated combinatorially into the appropriate positions of variable-region frameworks (Naturee Biotech. 18(2000) 852-6).

Gibbs M D et discloses a procedure for gene shuffling using degenerate primers that allows control of the relative levels of recombination between the genes that are shuffled (Gene 271(2001)13-20).

Zha D et al ChemBioChem 2003(4) 34-39 discloses a method for gene recombination based on appropriately prepared oligonucleotides.

Knappik A et al discloses.a consensus sequence that was derived for each of seven VH and seven VL germline families and optimized for expression in Escherichia coli (J. Mol. Biol.296(2000)57-86).

Jirholt P et al discloses antibody binding sites that provide an adaptable surface capable of interacting with essentially any molecular target. (Prot. Engin.14(2001)67-74).

Jirholt P et al discloses an approach in molecular design, where in vivo formed complementarity determining regions (CDR) from antibody genes are shuffled into a specific framework region. (Gene 215(1998)471-6).

WO 00/43507 A discloses an expression library comprising a repertoire of nucleic acid sequences cloned from a non-immunised source, each nucleic acid sequence encoding at least part of a variable domain of a heavy chain derived from an immunoglobulin naturally devoid of light chains (VHH).

In order to obtain meaningful products using DNA shuffling, particularly products that are different from the parental molecules, shuffling has to be performed between DNA molecules that share at least 70% homology. This limitation restricts the number of genes that may serve as templates as well as the range of diversity between the various templates and hence the resulting libraries posses a limited protein diversity and a limited range of improvement. Moreover, a comparison between DNA molecules of closely related genes from various organisms reveals that although at the amino acid level the peptides are quite similar, at the DNA level there is a very low sequence identity. Indeed, in evolution DNA tends to change much more rapidly than peptides by accumulation of silent and neutral mutations. Thus, the full potential of DNA shuffling as means to improve proteins can never be reached.

The significant contribution of template diversity to the diversity of the resulting library using DNA shuffling was demonstrated by Crameri et al. (Nature 391:288-291, 1998). Crameri et al. showed that using related genes from divergent natural sources as templates for DNA shuffling produces products with improved parameters that are 50 times better than the products obtained by the same method using templates from a single source that was manipulated in-vitro, since the range of diversity between the natural templates is in fact much wider than the range that may possibly be reached by the limited in vitro manipulation.

There remain considerable problems encountered with DNA shuffling as are known in the art, including the requirement for homology between the DNA templates, bias of the DNA shuffled products towards the parental DNA template (particularly those shuffled from divergent templates), and restricted diversity of the DNA shuffled products and to provide a simple system which enables extensive recombination between peptides in regions of peptide structure or amino acid similarity without constrains of DNA homology.

There is an unmet need for a system would enable the utilization of parental templates that cannot be used by current technologies, smaller but more divergent libraries will be produced, requiring fewer screening procedures, and the outcome would be products having greater improved qualities.

### SUMMARY OF THE INVENTION

It is an object of the present invention to provide recombinant chimeric proteins comprising a plurality of consensus amino acid regions corresponding to amino acid sequences or structures that are conserved in a plurality of related proteins. The recombinant chimeric proteins further comprise a plurality of variable regions corresponding to various amino acid sequences that are not necessarily conserved in said related proteins. The present invention further relates to methods for preparing the recombinant chimeric proteins and uses thereof that are less expensive, less work-intensive and more efficient than procedures used in current available methods. The advantage of the present invention is that shuffling between variable regions while maintaining the consensus backbone, increases the production of active enzymes while keeping high diversity, thereby, more favorable and important enzyme variants are generated. The related proteins may be derived from different organisms or from the same organism. The recombinant chimeric proteins may possess desired or advantageous characteristics such as lack of an unwanted activity and/or maintenance and even improvement of a desired property over the same property in the parental protein. The recombinant chimeric proteins can be selected by a suitable selection or screening method, wherein high throughput assays for detecting a new product is not essential, since typically the resulting recombinant chimeric proteins that show the desired activity or other required traits are significantly different from their parental templates derived from the related protein.

It is another object of the present invention to provide methods for generating designed libraries of recombinant chimeric proteins. In order to achieve the desired library the methods of the present invention comprise selection of a plurality of consensus regions which are conserved in a plurality of related proteins derived from different organisms and/or different proteins of the same organism. The methods further involve generation of a plurality of polynucleotides comprising, at their 5' and 3'-termini, uniform oligonucleotides capable of encoding the consensus regions and further comprising nucleotides capable of encoding variable regions corresponding to various amino acid sequences, which are not necessarily conserved in the related proteins. The methods further involve intentional recombination between the various uniform regions of the plurality of polynucleotides in order to form a plurality of chimeric polynucleotides. The present invention further relates to methods for preparing the recombinant chimeric proteins and uses thereof that are less expensive, less work-intensive and more efficient than procedures used in current available methods. The advantage of the present invention is that shuffling between variable regions that are not necessarily predetermined, while maintaining the consensus backbone, increases the production of active enzymes while keeping high diversity, thereby, more favorable and important enzyme variants are generated.

It is yet another object of the present invention to provide methods of using the recombinant chimeric proteins of the invention comprising formation of libraries of recombinant chimeric proteins or of chimeric polynucleotides, assays for screening libraries of recombinant chimeric proteins for various uses including searching for proteins with improved or preferred functionality, searching for ligands and receptors, among other uses and applications.

The methods of the present invention confer several significant advantages over methods known in the art for forming recombinant chimeric proteins or chimeric polynucleotides and for libraries thereof. One major advantage of the methods of the present invention is that it is explicitly not necessary to have any level of sequence homology other than that of the consensus region, between the polynucleotides used for recombination. Thus, the methods of the present invention are not limited by any natural homology barrier. The present invention enables utilization of screening procedures that are less work-intensive and less expensive to carry out than currently used methods. Due to constraints posed by homology in current methods, the parental enzymes have to be very similar to each other. As a result, although active chimeras are generated, these are not significantly different from their parents. Furthermore, screening for the chimeras produced by currently available methods usually require complex, quantitative high throughput assays. This problem is overcome in the present invention by the fact that shuffling is preferably performed between highly diverse parents, most of the products of such procedures are inactive, therefore, allowing easy quantitative screening or selection between inactive and active products even in high throughput systems to generate a second library of active products. The diverse nature of the active products of the present invention, thus leads to their properties also being diverse, thus making this library superior or better in terms of the potential to find a superior performing enzyme among its products. Therefore, a second, low-throughput but one that is highly specific screening for desired properties may be carried out in the present invention.

Use of the methods of the present invention is further advantageous as it results in the production of libraries with enhanced product diversity. This advantage is maintained even when the polynucleotides used for recombination confer a low sequence homology. The diverse nature of the active products of the present invention, thus leads to their properties also being diverse, thus making this library superior or better in terms of the potential to find a superior performing enzyme among its products. Therefore, a second, low-throughput but one that is highly specific screening for desired properties may be carried out in the present invention.

Furthermore, the libraries produced in accordance with the present invention do not exhibit a bias towards any product, and particularly are non-biased towards the parental related proteins. This is a significant advantage with respect to common methods of DNA shuffling. Using common methods of DNA shuffling as known in the art, with templates having significant non-homology between them, results mostly in parental-like polynucleotides since short polynucleotides that originate from the same parental template have a higher tendency to hybridize to each other, re-forming longer parental-like polynucleotides. Moreover, this tendency to produce parental-like products increases as the divergence between the starting polynucleotides increases. Since the resulting libraries contain mostly "noise", i.e. parental-like products, screening of the products is complicated, as it requires distinguishing between many products that are very similar to the parental templates. Thus, using the methods of the present invention it is possible to generate libraries of high divergence with a non-significant bias towards products that are similar to a parental template. Using the methods of the present invention it is further possible to dictate the prevalence of a given recombination product, or a given set of recombination products, by manipulating the molar ratio between the starting polynucleotides.

In addition, the methods and compositions of the present invention enable to obtain chimeric proteins comprising regions that are grossly non-conserved in a family of related as well as moderately related proteins.

Unlike known DNA shuffling methods, the present invention relies on highly induced recombination between short, specific, predefined regions. This approach is less dependent on polynucleotide sequence homology, and hence enables combination of regions of low polynucleotide sequence homology into the chimeric proteins.

According to a first aspect, the present invention provides a method for generating the recombinant chimeric proteins of the invention as set out in claim 1. An essential element of the methods of the present invention is the identification and selection of defined conserved amino acid regions within a plurality of preselected related proteins.

The term "related proteins" as used herein, refers to a plurality of proteins that are functionally- or structurally-related or to fragments of such proteins. The term as used herein is intended to include proteinaceous complexes, polypeptides and peptides, naturally occurring or artificial, wherein the former may be derived from the same organism or from different organisms.

In an embodiment, the consensus amino acid region is homologous to a segment of 3 to 30 amino acids, preferably 4 to 20 amino acids, more preferably 5 to 10 amino acids, that is conserved in the plurality of related proteins or fragments thereof.

In yet another embodiment, at least one consensus amino acid region is identical to a segment of 3 to 30 amino acids, preferably 4 to 20 amino acids, more preferably to 10 amino acids, derived from at least one of the related parental proteins or fragments thereof

According to various embodiments, the variable polynucleotide sequences comprised within the plurality of polynucleotides generated by the methods of the present invention, may posses less than 70% sequence homology, less than 50% sequence homology, less than 30% sequence homology and even less than 10% sequence homology.

In yet another embodiment, the variable polynucleotide sequences comprised within the plurality of polynucleotides generated by the methods of the present invention are substantially devoid of sequence homology.

In yet another embodiment, the recombination step is achieved in any suitable recombination system selected from the group consisting of: in vitro homologous recombination, in vitro sequence shuffling via amplification, in vivo homologous recombination and in vivo site-specific recombination.

In a certain embodiment, recombination is achieved by a method for assembling a plurality of DNA fragments comprising (a) providing a plurality of double stranded DNA fragments having at least one terminal single stranded overhang capable of encoding a consensus amino acid sequence, wherein the overhang terminus of each DNA fragment is complementary to the overhang of at least one other DNA fragment; and (b) mixing the DNA fragments under suitable conditions, to obtain recombination. The principles of this method are disclosed in US Patent No. 6,372,429 assigned to one of the inventors of the present invention.

In yet another embodiment, assembly of the recombined polynucleotides is achieved by a method selected from the group consisting of: ligation independent cloning, PCR, primer extension such as commonly used in DNA shuffling

In a preferred embodiment, the naturally occurring and non-natural polynucleotides from which the polynucleotides participating in the recombination are derived, are typically non related, particularly not by any sequence homology.

In yet another embodiment, the method of the present invention further comprises polynucleotide amplification prior to recombination.

In yet another embodiment, the method of the present invention comprises recombination between the plurality of polynucleotides in the presence of a plurality of vector fragments terminated at both ends with oligonucleotides that are complementary to any of the terminal sequences of any of said polynucleotides.

In yet another embodiment, the DNA is ligated into a vector prior to transforming the host cell.

In yet another embodiment, at least one of the clones formed by the method of the invention exhibits a specific enzymatic activity.

In yet another embodiment, the enzyme Uracil DNA Glycosylase (UDG) is added prior to the recombination step.

In yet another embodiment, the enzyme Uracil DNA Glycosylase (UDG) and N,N,dimethylethylenediamine are added prior to the recombination step.

In yet another embodiment, the enzyme Uracil DNA Glycosylase (UDG) and N,N,dimethylethylenediamine are added prior to the recombination step, following the addition of ligase at the step of recombination.

In yet another embodiment, the ratio between distinct polynucleotides at the recombination stage is selected from the group consisting of: an equimolar ratio, a non-equimolar ratio, a random ratio.

### BRIEF DESCRIPTION OF THE DRAWINGS

**Figure 1** shows five conserved amino acid regions (gray boxes), the consensus amino acid regions corresponding thereto and the consensus nucleic acid encoding thereof (below gray boxes), selected from a group of prokaryotic lipases by amino acid sequence alignment.
**Figure 2** represent an alignment of related amino acid sequence and identification of conserved regions (C.R.1 and C.R.2) of a similar structure and/or a similar amino acid sequence among non-conserved amino acid regions.
**Figure 3****:** is a scheme showing PCR amplification of a gene segments containing a "first" and a "second" PCR fragments sharing an overlap (1^{st} C.R; 2^{nd} C.R; 3^{rd} C.R. and last C.R.), with each other.
**Figure 4****:** is a scheme presenting exemplary combinatorial products (bottom) obtained from recombination between PCR fragments containing overlapping conserved regions (top)
**Figure 5****:** is a scheme describing a library of chimeric products (C) obtained from hybridization between overlapping regions of PCR fragments of related genes (A) by hybridization between the overlapping regions of the fragments following a single round of 5' to 3' extension of the single stranded strands (B).
**Figure 6****:** is a scheme describing assembly of PCR fragments of related genes, each PCR fragment contains overlapping conserved regions (A), assembly is generated by Ligation Independent Cloning (LIC) which includes conversion of the overlapping regions into single stranded overhangs (B) following formation of the assembled chimeric gene (C).
**Figure 7** demonstrates structural alignment between chitinaseA (1edq) and chitobiase (1qba). ***** - Regions of high structural similarity; B: beta sheet structure; H: alpha helix structure. The amino acid consensus sequence that is used in the beta sheet junctions is displayed below the alignment. The names of the specific primers that are used and their orientations are also displayed.

### DETAILED DESCRIPTION OF THE INVENTION

### Definitions

As used herein, "polynucleotide", "oligonucleotide" and "nucleic acid" include reference to both double stranded and single stranded DNA or RNA. The terms also refer to synthetically or recombinantly derived sequences essentially free of non-nucleic acid contamination. A polynucleotide can be a gene sub-sequence or a full length gene (cDNA or genomic). Unless specifically limited, the term encompasses nucleic acids containing known analogues of natural nucleotides, which have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605, 1985; Rossolini et al., Mol. Cell. Probes 8:91, 1994). The term nucleic acid is used interchangeably with gene, cDNA, and mRNA encoded by a gene.

The terms "polypeptide", "peptide" and "protein" are used interchangeably herein to refer to a polymer of amino acid residues. The terms include naturally occurring amino acid polymers and amino acid polymers in which one or more amino acid residue is an artificial chemical analogue of a corresponding naturally occurring amino acid.

The term "naturally-occurring" as used herein as applied to an amino acid or a polynucleotide that can be found in nature. For example, a polypeptide or polynucleotide sequence that is present in an organism that can be isolated from a source in nature and which has not been intentionally modified by man in the laboratory is naturally-occurring. Generally, the term naturally-occurring refers to an object as present in a non-pathological (undiseased) individual, such as would be typical for the species.

The term "conserved amino acid region" as used herein, refers to any amino acid sequence that shows a significant degree of sequence or structure homology in a plurality of related proteins.

A "significant degree of homology" is typically inferred by sequence comparison between two sequences over a significant portion of each of the sequences. In reference to conserved amino acid regions, a significant degree of homology intends to include at least 70% sequence similarity between two contiguous conserved regions within two distinct related proteins. A significant degree of homology further refers to conservative modifications including: individual substitutions, individual deletions or additions to a peptide, polypeptide, or a protein sequence, of a single amino acid or a small percentage of amino acids. Conservative amino acid substitutions refer to the interchange of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are described by the following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W). (see, e.g., Creighton, Proteins, 1984).

The term "consensus amino acid" refers to a uniform amino acid sequence corresponding to a distinct set of conserved amino acid regions derived from a plurality of related proteins, wherein the uniform amino acid confers a significant degree of homology to each conserved amino acid region of the set of conserved amino acid regions.

The term "uniform polynucleotide sequences" as used herein, refers to oligonucleotides, typically of 30-150 nucleotides, which are identical in a plurality of overlapping polynucleotides and are located at the termini of said overlapping polynucleotides. According to the present invention, there are two types of uniform polynucleotides, the first type is an oligonucleotide capable of encoding a consensus amino acid. The second type is an oligonucleotide which may encode any amino acid and not necessarily a conserved one. An example of the second type of uniform polynucleotide sequences would be the oligonucleotides at the termini of vector fragments and at the termini of the polynucleotides that are designed to recombine with the vector fragments.

The term "distinct polynucleotide" as used herein, refers to a polynucleotide that has a uniform polynucleotide sequence at each of its ends, enabling its recombination with other distinct polynucleotides, and a variable region in-between. It should be noted that the variable region may comprise three types: 1) predetermined sequences, 2) sequences that are determined in some regions and undetermined in others-such as sequences produced by error-prone PCR, and 3) sequences that are undetermined or scrambled-such as those produced by degenerate oligonucleotide sysnthesis.

The term "related proteins" or "a family of related proteins" are interchangeably used to describe a plurality of proteins that are functionally- or structurally- similar, or fragments of such proteins. The term as used herein is intended to include proteinaceous complexes, polypeptides and peptides, naturally occurring or artificial, wherein the former may be derived from the same organism or from different organisms. Functionally related proteins include proteins sharing a similar activity or capable of producing the same desired effect. Functionally related proteins may be naturally occurring proteins or modified proteins (with amino acid substitutions, both conservative and non-conservative) that have the same, similar, somewhat similar, modified activity as a wild-type or unmodified proteins. Structurally related proteins include proteins possessing one or more similar or identical particular structures, wherein each particular structure, irrespective of its amino acid sequence or with respect to its amino acid sequence, facilitates a particular role or activity, including binding specificity and the like.

The term "parental related proteins" or "parental proteins" as used herein, refer to the family or multiple families of related proteins which were utilized in a single recombination reaction. 5

Suitable "related proteins" of interest can be fragments, analogues, and derivatives of native or naturally occurring proteins. By ""fragment" is intended a protein consisting of only a part of the intact protein sequence and structure, and can be a C-terminal deletion or N-terminal deletion of the native protein or both. By "analogue" is intended an analogue of either the native protein or of a fragment thereof, where the analogue comprises a native protein sequence and structure having one or more amino acid substitutions, insertions, deletions, fusions, or truncations. Protein mimics are also encompassed by the term analogue. By "derivative" is intended any suitable modification of the native protein of interest, of a fragment of the native protein, or of their respective analogues, such as glycosylation, phosphorylation, or other addition of foreign moieties, so long as the desired activity of the native protein is retained.

The term "wild-type" means that the amino acid fragment does not comprise any mutations. A "wild-type" protein means that the protein will be active at a level of activity found in nature and typically will comprise the amino acid sequence found in nature. In an aspect, the term "wild type" or "parental sequence" can further indicate a starting or reference sequence prior to a manipulation of the invention.

In the polypeptide notation used herein, the left-hand direction is the amino terminal direction and the right-hand direction is the carboxy-terminal direction, in accordance with standard usage and convention. Similarly, unless specified otherwise, the left-hand end of single-stranded polynucleotide sequences is the 5' end; the left-hand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the ADA and which are 3' to the 3' end of the coding RNA transcript are referred to as "downstream sequences".

As used herein "protein library" refers to a set of polynucleotide sequences that encodes a set of proteins, and to the set of proteins encoded by those polynucleotide sequences, as well as the fusion proteins containing those proteins.

### Preferred modes for carrying out the invention

The present invention provides methods and compositions enabling extensive recombination between polynucleotides encoding peptides and polypeptide fragments derived from proteins having a common function and/or a common structure without constrains of DNA homology.

According to a particular embodiment of the present invention, a method for generating a plurality of recombinant chimeric proteins is provided. The method comprise, as an essential feature, selection of a plurality of consensus amino acid sequences, such that each consensus amino acid sequence corresponds to a distinct amino acid sequence that is conserved in a plurality of related proteins. The conserved amino acid regions may correspond to conserved amino acid sequences or to conserved amino acid structures, such as conserved peptide structures. Certain aspects of the invention integrate both types of conserved regions. In a certain embodiments, the selected conserved amino acid regions are short, and protein function is not abolished upon their exchange with a designed consensus sequence.

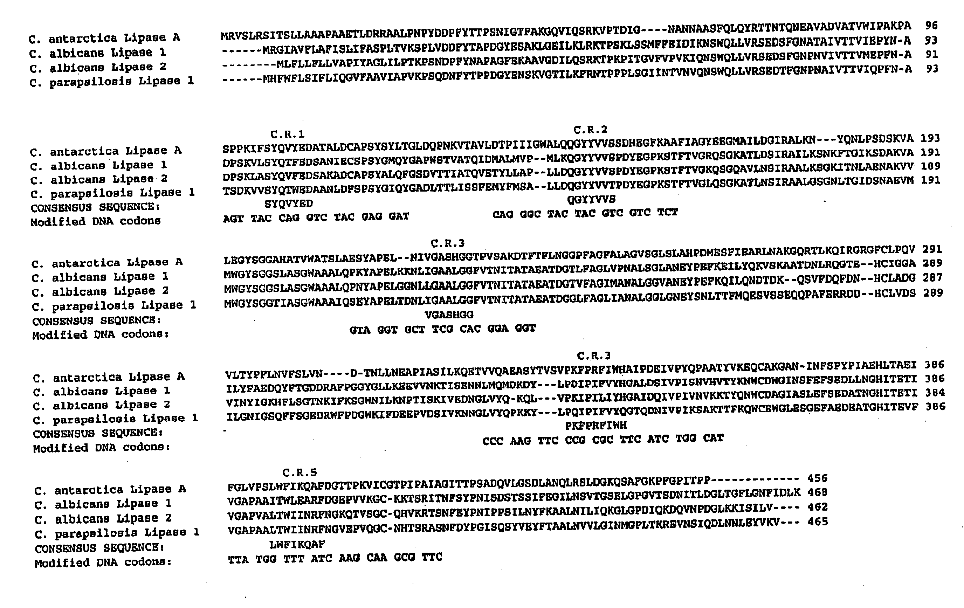

Identification of conserved amino acid regions is typically performed through amino acid sequence alignment of a plurality of proteins (Fig. 1). The plurality of proteins may be randomly selected and following a preliminary amino acid sequence alignment, the randomly selected proteins are divided into groups of related proteins, such that the member proteins in each group posses a particular range of amino acid sequence similarity. Alternatively, the plurality of proteins utilized to identify conserved amino acid regions may be deliberately selected from a group of proteins known to posses a specific activity, a certain structure or both. The proteins or peptides may be derived from different microorganisms or from different proteins and proteinaceous complexes (e.g. the cellulosome) of the same organism.

Amino acid sequence alignment is usually conducted using any protein search tool, which allows to input protein sequences and to compare these against other protein sequences, such as Protein BLAST. The proteins are selected from protein databases, wherein search for related protein is conducted in protein databases, protein structure databases and conserved domains databases among others.

Following identification of a plurality of conserved amino acid regions in a plurality of related proteins, a consensus amino acid sequence is determined for each distinct conserved amino acid region. A distinct conserved amino acid region is generally a set of a plurality of regions, being conserved in the plurality of related proteins. Accordingly, each consensus amino acid region confers a significant similarity to each conserved region of a distinct set of conserved amino acid regions, wherein the consensus sequence is of 3 to 30 amino acids, preferably 4 to 20 amino acids, more preferably 5 to 10 amino acids.

Distinct polynucleotides are produced once a plurality of conserved regions are identified in a plurality of parental proteins, and consensus amino acid regions are determined, wherein the parental proteins are a family of related proteins or multiple families of related proteins. Each consensus amino acid sequence corresponds to a conserved amino acid sequence or a conserved amino acid structure in a group of related proteins. Accordingly, a typical polynucleotide, also termed hereinafter "an overlapping polynucleotide", comprises a gene encoding any fragment of the related proteins, also termed herein "a variable region", and is further terminated at least on one side with distinct terminal oligonucleotide sequences capable of encoding a consensus amino acid sequence. Each overlapping polynucleotide may further comprise a terminal uniform oligonucleotide, which does not encode a consensus amino acid sequence but overlaps with at least another distinct polynucleotides within the compositions of the invention. The variable regions of the plurality of polynucleotides generated by the methods of the present invention or comprised within the compositions of the present invention may exhibit a reduced level of sequence homology, less than 70% sequence homology, less than 50% sequence homology, less than 30% sequence homology and even less than 10% sequence homology. The present invention further relates to methods for preparing the recombinant chimeric proteins and uses thereof, that are less expensive, less labor-intensive and more efficient than procedures that are used currently. The advantage of the present invention is that by shuffling between variable regions while maintaining the consensus backbone, the production of active enzymes with high diversity , is increased.

It should be noted that the DNA shuffling approaches known in the art mainly depend on random recombination between randomly fragmented polynucleotides. As these processes rely on cross hybridization between contiguous nucleotides and since the hybridization depends on homology, fragmented polynucleotides derived from a given relatively long parental polynucleotide tend to hybridize to polynucleotide fragments that are highly complementary (homologous) rather than to hybridize with fragments that are not highly complementary. Thus, short regions of homology shared between the various fragmented polynucleotides do not generate new extension products and the final hybridization products are primarily similar or identical to the parental polynucleotide. This is true even in cases where homology between the parental types is quite high and deliberate attempts are made to encourage such recombination (e.g. US Pat No. 6,479,652). The occurrence of double or triple recombinants in such cases is even more rare.

The present invention enables utilization of screening procedures that are less labor-intensive and more cost-effective than procedures currently in use. Due to the constraints posed by homology in current methods, the parental enzymes have to be very similar to each other. As a result, active chimeras are generated, but these are not significantly diverse from their parents. Furthermore, screening for the chimeras produced by current methods usually requires complex quantitative high throughput assays. This problem is overcome by the present invention by shuffling between variable regions while keeping the conserved regions unaffected. This ensures production of improved and high rates of active products.

Use of the methods of the present invention is further advantageous as it results in the production of libraries with enhanced product diversity. This advantage is maintained even when the polynucleotides used for recombination confer a low sequence homology. Furthermore, since shuffling between variable regions is preferably performed between highly diverse parents, most of the products of such procedures are inactive, and therefore, allow easy quantitative screening or selection between inactive and active products even in high throughput systems to generate a second library of active products. The diverse nature of the active products of the present invention thus leads to more diverse properties and thus a better or superior library in terms of the potential to fmd better performing enzymes among its products. Therefore, a second screen that is a low-throughput but highly specific assay for desired properties may be carried out in the present invention.

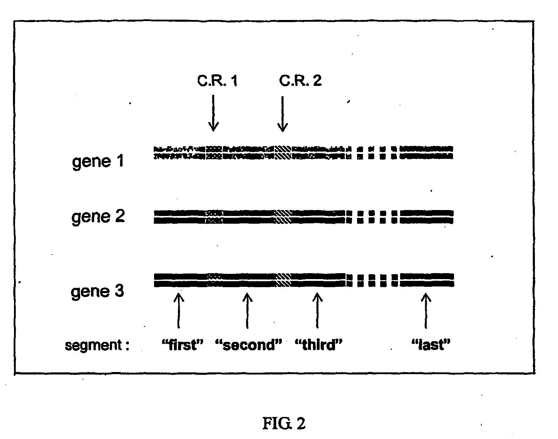

Typically, a first distinct overlapping polynucleotide has a downstream terminal sequence which is identical to the upstream terminal sequence of a second distinct polynucleotide (Fig. 2), the downstream terminal sequence of the second distinct polynucleotide is identical to the upstream terminal sequence of a third distinct polynucleotide, and so on.

According to a preferred embodiment, the distinct polynucleotides of the methods and compositions of the present invention are produced by PCR using appropriate primers, wherein the appropriate primers comprise the following elements: a 5' portion which is identical to a uniform oligonucleotides encoding a consensus amino acid sequences; at least one dU nucleotide replacing one or more of the dT nucleotides of the uniform sequence, wherein the replaced dT is within the 10 to 30 nucleotides from the 5' terminus of the primer; a 3' terminus that is complementary to a gene fraction encoding a fragment of a desired parental protein. The source from which the distinct polynucleotides are isolated or the variable polynucleotides therein may be any suitable source, for example, from plasmids such a pBR322, from cloned DNA or RNA or from natural DNA or RNA from any source including bacteria, yeast, viruses and higher organisms such as protozoa, fungi, plants or animals. DNA or RNA may be extracted from blood or tissue material. The template polynucleotide may be obtained by amplification using the polynucleotide chain reaction (PCR) (U.S. Pat. Nos. 4,683,202 and 4,683,195). The polynucleotide may be present in a vector present in a cell and sufficient nucleic acid may be obtained by transforming the vector into a cell, culturing the cell and extracting the nucleic acid from the cell by methods known in the art.

The plurality of distinct polynucleotides may be amplified prior to recombination to obtain distinct sets of polynucleotides using amplification methods known in the art, commonly using PCR reaction (U.S. Pat. Nos. 4,683,202 and 4,683,195) or other amplification or cloning methods. However, the removal of free primers from the PCR products before hybridization provides a more efficient result. Removal of free primers from the composition may be achieved by numerous methods known in the art including forcing the composition through a membrane of a suitable cutoff by centrifugation.

The plurality of distinct polynucleotides are mixed randomly or mixed using a predetermined prevalence of the plurality of distinct polynucleotides, to form a composition of overlapping polynucleotidesencourage atconsensus/uniform/ is encouraged. The composition comprises distinct polynucleotides derived from a single family of related proteins and preferably comprises distinct polynucleotides derived from multiple families of related proteins. The number of distinct polynucleotides in a composition is at least about 25, preferably at least about 50, preferably at least about 100 and more preferably at least about 500.

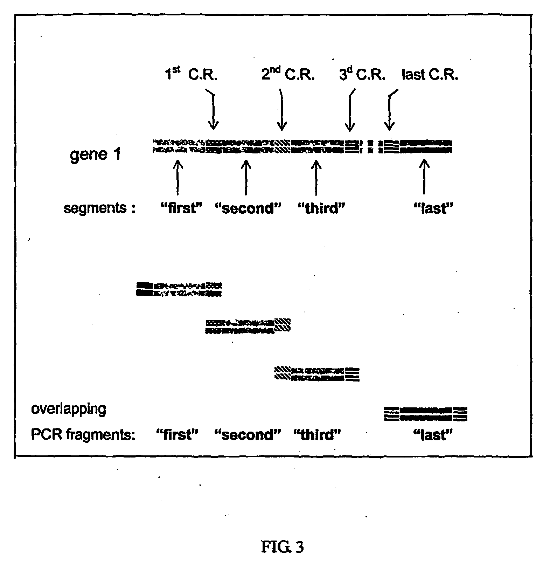

The composition of overlapping polynucleotides may be maintained under conditions which allow hybridization and recombination of the polynucleotides and generation of a library of chimeric polynucleotides (Fig. 3). It is contemplated that multiple families of related proteins may be used to generate a library of chimeric polynucleotides according to the method of the present invention, and in fact were successfully used.

The optimal conditions for hybridization, also termed "stringent conditions" or "stringency", refer to the conditions for hybridization as defined by the nucleic acid, salt, and temperature and are well known in the art. Numerous equivalent conditions comprising either low or high stringency depend on factors such as the length and nature of the sequence (DNA, RNA, base composition), nature of the target (DNA, RNA, base composition), milieu (in solution or immobilized on a solid substrate), concentration of salts and other components (e.g., formamide, dextran sulfate and/or polyethylene glycol), and temperature of the reactions (within a range from about 5°C to about 25°C below the melting temperature of the probe). One or more factors may be varied to generate conditions of either low or high stringency while only those single-stranded overlapping polynucleotides having regions of homology with other single-stranded overlapping polynucleotides will undergo hybridization to form double stranded segments. For example, a slow cooling of the temperature could provide a suitable temperature gradient such that each distinct single stranded overhangs will undergo hybridization at an appropriate temperature within the provided temperature gradient.

Recombination step may be achieved by any suitable recombination system selected from the group consisting of: in vitro homologous recombination, in vitro sequence shuffling via amplification, in vivo homologous recombination and in vivo site-specific recombination.

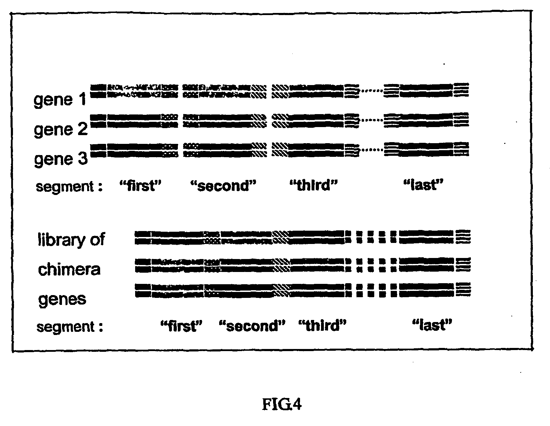

According to another preferred embodiment, hybridization and recombination of the distinct polynucleotides may be performed by a single round of primer extension (Fig. 4). Two distinct polynucleotides hybridize through their overlapping uniform sequences, wherein at least one overlapping uniform sequence of each overlapping polynucleotide may correspond to a consensus amino acid sequence. Following hybridization, extension of the single stranded 5' and 3' overhangs, takes place. Filling-in of single stranded locations within the double stranded assembled chimeric polynucleotide is optionally performed in vitro in the presence of DNA polymerase, dNTPs and ligase. This method differs from PCR, in that the number of the polymerase start sites and the number of molecules remains essentially the same wherein in PCR, the number of molecules grows exponentially.

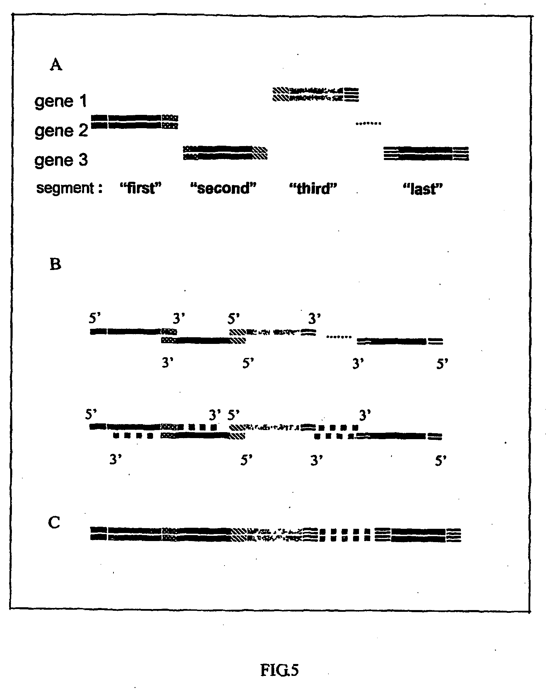

According to an additional preferred embodiment of the invention, following hybridization the overlapping terminals of the double stranded polynucleotides are converted into long single-stranded overhangs. According to this embodiment, the fragments are then connected to each other and cloned by Ligation Independent Cloning (LIC) procedure (Fig. 5).

According to yet another embodiment, hybridization and recombination of the overlapping polynucleotides is performed in-vivo. According to this embodiment, host cells are transfected with the composition of the overlapping polynucleotides and recombination is performed by the endogenous recombination machinery of the host.

According to a further embodiment of the invention, the overlapping polynucleotides of the composition may comprise sequences that are not related to the parental proteins or to the consensus sequences.

The molar ratio of the distinct overlapping polynucleotides in the composition of the present invention may be equimolar between all distinct polynucleotides (1:1:1...:1) or other ratio that is suitable to promote the recombination of a specific library of chimeric polynucleotides.

The length of distinct polynucleotides may vary from overlapping polynucleotide sequences containing more than 20 nucleotides to overlapping polynucleotide sequences containing more than 100 nucleotides, more than 400 nucleotides, more than 1000 nucleotides. Preferably, the length of overlapping polynucleotides is more than 20 nucleotides and not more than 5000 nucleotides, preferably, the length of an overlapping polynucleotides is between about 100 to about 400 nucleotides.

According to one preferred embodiment of the methods and compositions of the present invention, a polynucleotides which is designed to overlap with a vector fragment comprises a common uniform terminal sequence located upstream or downstream of the beginning or termination of the coding region of said overlapping polynucleotide. At the end of recombination in the presence of vector fragments, such polynucleotides will be at the termini of the resulting chimeric genes and will 'stick' to the vector fragments.

Recombination may be further achieved by a method for assembling a plurality of overlapping polynucleotides, comprising (a) providing a plurality of double stranded DNA fragments having at least one terminal single stranded overhang capable of encoding a consensus amino acid sequence, wherein the overhang terminus of each DNA fragment is complementary to the overhang of at least one other DNA fragment; and (b) mixing the DNA fragments under suitable conditions, to obtain recombination. The principles of this method are disclosed in US Patent No. 6,372,429 assigned to one of the inventors of the present invention.

Preferably, the enzyme Uracil DNA Glycosylase (UDG) and N,N,dimethylethylenediamine (DMED) are added to the composition of overlapping polynucleotides prior to recombination (US 6,372,429). UDG specifically depirimidinates deoxyuracil nucleotides, and DMED forms a nick just 3' of a-pirimidinated residues.

Recombination between the plurality of polynucleotides may be performed in the presence of a plurality of vector fragments terminated at both ends with single stranded overhangs that are complementary to any of the terminal sequences of any of said polynucleotides. Alternatively, the library of chimeric polynucleotides is ligated into a plurality of vectors prior to transfection of a plurality of host cells. For this purpose any vector may be used for cloning provided that it will accept a chimeric polynucleotide of the desired size.

For expression of the chimeric polynucleotide, the cloning vehicle should further comprise transcription and translation signals next to the site of insertion of the DNA fragment to allow expression of the chimeric polynucleotide in the host cell. The vector may comprises at least one additional component selected from the group consisting of a restriction enzyme site, a selection marker gene, an element capable of regulating production of a detectable enzymatic activity, an element necessary for propagation and maintenance of vectors within cells. The vector is selected from the group consisting of: a plasmid a cosmid, a YAC, a BAC, or a virus. Expression vectors containing all the necessary elements for expression are commercially available and known to those skilled in the art. See, e.g., Sambrook et al., Molecular Cloning: A Laboratory Manual, Second Edition, Cold Spring Harbor Laboratory Press, 1989. Preferred vectors include the pUC plasmid series, the pBR series, the pQE series (Quiagen), the pIRES series (Clontech), pHB6, pVB6, pHM6 and pVM6 (Roche), among others.

A plurality of host cells is transfected with the library of chimeric polynucleotides of the invention, for maintenance and for expression of a corresponding library of chimeric proteins. To permit the expression of the library of chimeric polynucleotides in the host cells the chimeric polynucleotides are placed under operable control of transcriptional elements. Upon transfection, a library of cloned cell lines is obtained. The clones may be cultured utilizing conditions suitable for the recovery of the protein library from the cloned cell lines. At least one of the clones may exhibit a specific enzymatic activity. This mixed clone population may be tested to identify a desired recombinant protein or polynucleotide. The method of selection will depend on the protein or polynucleotide desired. For example, if a protein with increased binding efficiency to a ligand is desired, the clone library or the chimeric polypeptide library reconstructed therefrom may be tested for their ability to bind to the ligand by methods known in the art (i.e. panning, affinity chromatography). If a protein with increased drug resistance is desired, the protein library may be tested for their ability to confer drug resistance to the host organism. One skilled in the art, given knowledge of the desired protein, could readily test the population to identify the clone or the chimeric protein which confer the desired properties.

It is contemplated that one skilled in the art could use a phage display system in which fragments of the recombinant chimeric proteins of the invention are expressed as fusion proteins on the phage surface (Pharmacia, Milwaukee Wis.). The recombinant chimeric polynucleotides are cloned into the phage DNA at a site, which results in the transcription of a fusion protein, a portion of which is encoded by the recombinant chimeric polynucleotide. The phage containing the recombinant nucleic acid molecule undergoes replication and transcription in a host cell. The leader sequence of the fusion protein directs the transport of the fusion protein to the tip of the phage particle. Thus the fusion protein, which is partially encoded by the recombinant chimeric polynucleotides, is displayed on the phage particle for detection and selection by the methods described-above. In this manner, recombinant chimeric proteins with even higher binding affinities or enzymatic activity, than that conferred by the parental proteins or other known wild-type proteins, could be achieved.

According to a third aspect, the present invention provides recombinant chimeric proteins comprising a plurality of consensus amino acid regions corresponding to amino acid sequences that are conserved in a plurality of related proteins. The recombinant chimeric proteins further comprise a plurality of variable regions corresponding to various amino acid sequences derived from the related proteins. Said variable regions may be deliberately selected and included in the chimeric products for the purposes of designing vaccines and synthetic antibodies.

It is a fourth aspect of the present invention to provide methods of using the recombinant chimeric proteins of the invention comprising formation of libraries of chimeric proteins and libraries of chimeric genes, providing assays for screening libraries of recombinant chimeric proteins for various uses including searching for proteins with improved or preferred functionality, searching for ligands and receptors, among other uses and applications.

### EXAMPLES:

### EXAMPLE 1. CREATION OF A CHIMERA LIPASE LIBRARY

Lipases are the most widely used enzymes for industrial purposes. The methods of the present invention were utilized to create a lipase library encoding proteins with new traits. Clones expressing individual proteins from this library are screened for improved industrial traits. The library construction was based on the following parental genes, encoding four lipase genes: Lipase A from *Candida Antarctica,* Lipase 1 and Lipase 4 from *Candida albicans,* and Lipase 1 from *Candida prapsilosis.* These genes share very little DNA homology but exhibit notable protein similarity. Protein similarity search was carried out by BLAST (http://www.ncbi.nlm.nih.gov/BLAST) with *C*. *Antarctica* Lipase A as the original protein sequence.

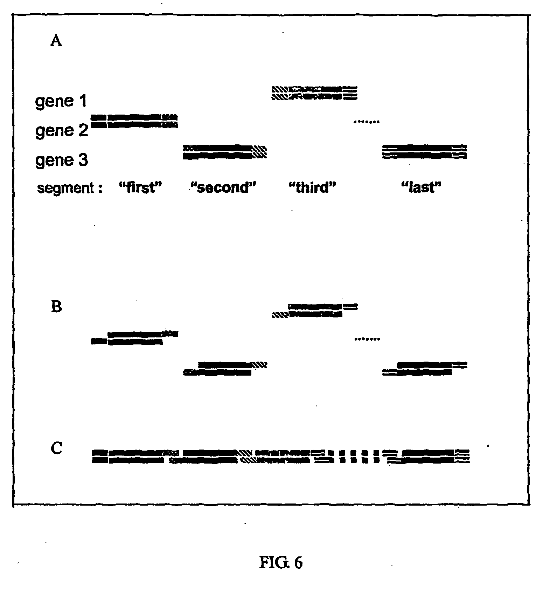

Computer aided alignment of these proteins was conducted using ClustalW peptide alignment software (Thompson JD et al., 1994, Nucleic Acids Res. 22: 4673). The alignment revealed some degree of amino-acid homology, and even a higher degree of amino acid similarity between them (Figure 6). It is estimated that the more conserved regions are the most essential for protein function whereas the less conserved are regions that have been used throughout evolution to search for the modifications that have contributed to the typical performance characteristics of the various proteins. Thus, a library of recombinant chimeric protein variants was prepared, such that less conserved regions were replaced by the analogous regions from similar proteins from the same organism (Lipase 1 and 4 from *C*. *albicans)* or other organisms (*C. Antarctica* and *C. parapsilosis*), while at the same time, the conserved regions remained unchanged.

Five short conserved regions, 7-8 amino acids long were identified (Fig. 6, gray boxes). A consensus amino acid sequence was designed for each conserved region, and uniform DNA sequences encoding the designed consensus regions were further determined (Fig. 6, displayed below each gray box). The codons of the each given consensus amino acid sequence were modified such that they would be identical in all parental genes. The choice for the codon usage was made using Oligo software (Oligo^{R} Primer Analysis, version 5 for PC, National Biosciences Inc. 3650 Annapolis Lane North, #140 Plymouth, MN 55447-5434) in order to minimize the possible formation of single-strand "stem-loop" structures that might decrease the ability of segments to inter-connect.

DNA fragments comprising overlaps of the conserved regions, were produced by PCR. The conserved overlaps were generated by using primers (Sigma, Israel) containing two essential parts: the first part is a sequence of 20-30 bases at the 5' end of the primer that is complementary to 20-30 bases at the end of the following DNA fragment. This part included two dU nucleotides, which replaced dT nucleotides. The second part was the 3' end of the primer containing a sequence which is complementary to the template DNA and enables the initiation of the PCR process. The following primers were used to generate a linear pYES2 vector with ends that are complementary to the various lipases (Table 1): #CALAupper (SEQ ID NO. 1) and #CALA1ow (SEQ ID NO. 2), #Lip1upper (SEQ ID NO. 3) and #Lip11ow (SEQ ID NO. 4), #Lip4upper (SEQ ID NO. 5) and #Lip41ow (SEQ ID NO. 6), #PLip1upper (SEQ ID NO. 7) and #PLip11ow (SEQ ID NO. 8), respectively. The resulting PCR fragments were subcloned, into the linearized pYES2 expression vector (Invitrogen) that was PCR amplified using primers #VecMat (SEQ ID NO. 9) and #VecTerm (SEQ ID NO. 10). The vector fragment contained a bacterial origin of replication, a yeast origin of replication, two selectable markers: a bacterial one (Ampicilin resistance gene) and a yeast marker (URA3), a strong alpha mating type promoter coupled with a secretion leader sequence. In addition, the vector segment contained two long overhangs at its ends: The first overhang, at one end of the segment, was at the end of the secretion leader sequence. This overhang was complementary to the overhang of the "first" PCR fragment. The second overhang, at the other end of the segment, was situated next to a Cytochrome C terminator sequence. This overhang was complementary to the overhang of the last PCR fragment.

**Table 1:**

| Primer name | Sequence | Junction | SEQ ID NO. | Direction |
|---|---|---|---|---|
| #CALAupper | AAGGGGUACCTTTGGAUAAAAGACTGGACCGACGGGCGGCGCT | Vec.upstream | 1 | forward |
| #Lip1upper | AAGGGGUACCTTTGGAUAAAAGATCCCCCTTGACTGTAAAGTC | vec. upstream | 3 | forward |
| #Lip4upper | AGGGGUACCTTTGGAUAAAAGAGGTCTTATTTTACCAACTAA | vec. upstream | 5 | forward |
| #PLip1upper | AAGGGGUACCTTTGGAUAAAAGAGCCGTCATTGCCCCAGTTAA | vec. upstream | 7 | forward |
| #1uLipcala | ATCCTCGUAGACCTGGTAACUAAAGATCTTGGGCGGCGAAGC | C.R.1 | 11 | forward |
| #1ulip1 | ATCCTCGUAGACCTGGTAACUAAGGACTTTGGATGGGTCAGC | C.R.1 | 12 | forward |
| #1uLip4 | ATCCTCGUAGACCTGGTAACUAGCGAGTTTGGAAGGGTCAGC | C.R.1 | 13 | forward |
| #1uPlip1 | ATCCTCGUAGACCTGGTAACUCACCACCTTGTCAGAAGTTGC | C.R.1 | 14 | forward |
| i1dLipca1a | AGTTACCAGGUCTACGAGGAUGCCACGGCGCTCGACTGTGCTCC | C.R.1 | 15 | reverse |
| #1dLip1 | AGTTACCAGGUCTACGAGGAUTCTGCAAATATTGAATGTTCACC | C.R.1 | 16 | reverse |
| #1dLip4 | AGTTACCAGGUCTACGAGGAUTCCGCTAAAGCTGATTGTGCCCC | C.R.1 | 17 | reverse |
| #1dPlip1 | AGTTACGAGGUCTACGAGGAUGCAGCAAACTTGGATTTTTCACC | C.R.1 | 18 | reverse |
| #2uLipcala | AGAGACGACGUAGTAGCCCUGCTGCAGCGCCCAGCCGATGATGA | C.R.2 | 19 | forward |
| #2tulip1 | AGAGACGACGUAGTAGCCCUGCTTCAACATAGGTACCATTAAAG | C.R.2 | 20 | forward |
| #2uLip4 | AGAGACGACGUAGTAGCCCUGGTCCAATAATGGTGCCAATAAAT | C.R.2 | 21 | forward |
| #2uPlip1 | AGAGACGACGUAGTAGCCCUGATCCAACAATGCTGACATAAAAT | C.R.2 | 22 | forward |
| #2dLipcala | AGGGCTACUACGTCGTCTCUTCCGACCACGAAGGCTTCAA | C.R.2 | 23 | reverse |
| #2dip1 | AGGGCTACUACGTCGTCTCUCCTGATTATGAGGGACCAAA | C.R.2 | 24 | reverse |
| #2dLip4 | AGGGCTACUACGTCGTCTCUCCAGACTATGAAGGTCCTAA | C.R.2 | 25 | ceverse |
| #2dPlip1 | AGGGCTACUACGTCGTCTCUCCCGATTACGAAGGTCCAAA | C.R.2 | 26 | reverse |
| #3uLipcala | ACCTCCGUGCGAAGCTCCUACAATGTTGAGCTCGGGCGCGTA | C.R.3 | 27 | forward |
| #3uLip1 | ACCTCCGUGCGAAGCTCCUACCAAATTTTTCTTCAATTCTGG | C.R.3 | 28 | forward |
| #3uLip4 | ACCTCCGUGCGAAGCTCCUACTAAGTTACCACCCAATTCTGG | C.R.3 | 29 | forward |
| #3uPlip1 | ACCTCCGUGCGAAGCTCCUACCAAATTGTCAGTCAATTCTGG | C.R.3 | 30 | forward |
| #3dLipcala | AGGAGCTUCGCACGGAGGUACGCCAGTGAGCGCCAAGGAC | C.R.3 | 31 | reverse |
| #3dLip1 | AGGAGCTUCGCACGGAGGUTTTGTCACTAATATTACTGCC | C.R.3 | 32 | reverse |
| #3dLip4 | AGGAGCTUCGCACGGAGGUTTTGTTACTAATATTACTGCT | C.R.3 | 33 | reverse |
| #3dPlip1 | AGGAGCTUCGCACGGAGGUTTTGTCACAAATATAACGGCT | C.R.3 | 34 | reverse |
| #4uLipcala | ATGCCAGAUGAAGCGCGGGAACUTGGGCACCGATACCGTGTAGCTCG | C.R.4 | 35 | forward |
| #4uLip1 | ATGCCAGAUGAAGCGCGGGAACUTGGGTAAATAATCTTTATCCATTT | C.R.4 | 36 | forward |
| #4uLip4 | ATGCCAGAUGAAGCGCGGGAACUTGGGAACTAGCTGTTTCTGATAAA | C.R.4 | 37 | forward |
| #4uPlip1 | ATGCCAGAUGAAGCGCGGGAACUTGGGAAGATATTTTTTCGGTTGAT | C.R.4 | 38 | forward |
| #4dLipcala | AGTTCCCGCGCUTCATCTGGCAUGCGATCCCCGACGAGATCGT | C.R.4 | 39 | reverse |
| &4dLip1 | AGTTCCCGCGCUTCATCTGGCAUGGTGCATTGGATTCCATTGT | C.R.4 | 40 | reverse |
| #4dLip4 | AGTTCCCGCGCUTCATCTGGCAUGGTGCTATTGACCAAATTGT | C.R.4 | 41 | reverse |
| #4dPlip1 | AGTTCCCGCGCUTCATCTGGCAUGGAACTCAGGATAATATTGT | C.R.4 | 42 | reverse |
| #5uLipcala | AGGCTTGCTUGATAAACCAUAAGCTAGGCACCAGACCAAAGA | C.R.5 | 43 | forward |
| #5uLip1 | AGGCTTGCTUGATAAACCAUAAAGCTGCCGGGGCCCCAACAA | C.R.5 | 44 | forward |
| #5uLip4 | AGGCTTGCTUGATAAACCAUAAAGCAACAGGAGCGCCAACAA | C.R.5 | 45 | Forward |
| #5uPlip1 | AGGCTTGCTUGATAAACCAUAAAGCAGCCGGCGCCCCGACAA | C.R.5 | 46 | forward |
| #5dLipcala | ATGGTTTAUCAAGCAAGCCUTCGACGGCACCACACCCAAGGT | C.R.5 | 47 | reverse |
| #5dLip1 | ATGGTTTAUCAAGCAAGCCUTCGACGGTGAACCAGTCGTCAA | C.R.5 | 48 | reverse |
| #5dLip4 | ATGGTTTAUCAAGCAAGCCUTCAATGGAAAACAAACCGTGTC | C.R.5 | 49 | reverse |
| #5dPlip1 | ATGGTTTAUCAAGCAAGCCUTCAATGGAGTTGAGCCTGTTCA | C.R.5 | 50 | reverse |
| #CALAlow | AAGAAGUCCAAAGCTTCAGCUCTAAGGTGGTGTGATGGGGCC | vec. downstream | 2 | reverse |
| #Lipllow | AAGAAGUCCAAAGCTTCAGCUCTATTTTAAGTCAATAAAGTT | vec. downstream | 4 | reverse |
| #Lip41ow | AAGAAGUCCAAAGCTTCAGCUCTATACAAGTATTGAAATCTT | vec. downstream | 6 | reverse |
| #Plipllow | AAGAAGUCCAAAGCTTCAGCUTTACACTTTTACATACTCTAA | vec. downstream | 8 | reverse |
| #VecMat | ATCCAAAGGUACCCCTUCTTCTT(linear vector) | vec. upstream | 9 | reverse |
| #VecTerm | AGCTGAAGCTUTGGACTTCTUCG(linear vector) | vec. downstream | 10 | forward |

A Stratagene-Robocycler gradient 96 thermocycler was used to calibrate the annealing temperatures. A Techne-Genius thermocycler was then used to amplify the various fragments and the linear vector. Taq DNA Polymerase (Gibco-BRL Life-Technologies) and 10x PCR Buffer were added together with dNTPs, template and primers to the PCR reaction mixture. Using a preparative TAE agarose gel, each required fragment (or linear vector) was separated from a 400µl reaction volume. The resulting PCR segments contained: A first segment from Lipase A of *C*. *Antarctica* that shares homology at its upstream end with one end of the linearized vector and homology with the first conserved region (C.R.1; Fig. 6) at its downstream end; A second segment from Lipase A that shares homology at its upstream end with C.R.1 and with C.R.2 at its downstream end; A third segment from Lipase A that shares homology at its upstream end with C.R.2 and with C.R.3 at its downstream end; A fourth, fifth and sixth segments that correspond to portions of Lipase A of *C*. *Antarctica* were created in the same way. Likewise, PCR segments corresponding to the other lipase genes were created in the same way. The regions corresponding to the subcloning junctions, as well as to C.R.1, C.R.2, C.R.3, C.R.4, and C.R.5, in all the segments were changed, at the time of oligonucleotide synthesis such that all would be homologous and would encode the respective amino acids of the *C*. *Antarctica* conserved regions in order not to disrupt inter-conserved region interactions, in case such interactions exist. Fragments then were purified with the QIAquick Gel Extraction kit (QIAGEN), according to manufacturer instructions. Pharmacia-Biotech Ultrospec 2000 spectrophotometer was used to determine the DNA concentration of the fragments and of the vector.

Equimolar amounts of DNA fragments and vector were used. The overhangs of the linear vector and of the DNA fragments for the construction of the inserts were exposed as follows: a) UDG enzyme was used to a-pyrimidinate the dU nucleotides (Nisson et al., 1991, PCR Methods Appl., 1:120-123); b) N,N,Dimethyl-ethylenediamine was used to nick the a-pyrimidinic dU nucleotides (McHugh et al., 1995, Nucleic Acids Res., 23:1664; c) The reaction mixture was heated to 70°C for 5 minutes and then purified using the QIAquick PCR Purification Kit (QIAGEN) to get rid of the short oligonucleotides created by overhang exposure. The DNA fragments were eluted in 20mM Tris-HCl pH 8.4, 50mM KCl,1.5mM MgCl₂.

Assembly was performed by heating the purified mixture to 70°C and allowing slowly cooling from 70°C to 37°C, although hydrogen bonds are weak, overhangs of 20-30 nucleotides are extremely stable as described in US 6,372,429.

For transformation, a BioRad E. coli pulser was use. The electroporation processes were performed according to well-known procedures. Transformed cells were transferred to LB Amp plates and individual colonies were isolated and analyzed by restriction digestion.

### EXAMPLE 2. CREATION OF A CHIMERIC POLYPEPTIDE LIBRARY

To demonstrate that the present invention is not restricted to specific protein families and not even to proteins, a library of plasmids that contain constructs made of three variable regions A, B and C, located between four short conservative regions was designed. Two alternative sequences were assigned to each region. The sequences were designed such that they differed in content and length (Table 2). Sequence A1 from region A is 100bp in length and encodes a portion of the Tet^{r} protein from pBR322. In contrast, sequence A2 is 150bp in length and encodes a non protein sequence. Likewise, sequence B1 and B2 are 100bp and 200bp respectively, encoding an internal portion and the carboxy terminus of the Tet^{r} protein. Sequences C1 and C2 are 150 and 350bp, encoding a portion of ROP, a mediator of RNA I activity from pBR322, and a portion of Amp^{r} from pBR322, respectively. The conserved regions consisted of a short sequence (ATGTTCTTTCCTGCGTTCAGGCGT; SEQ ID NO. 51), which separates between regions A and B, another conserved sequence (AATCAGCTAGTGACTGGACGAT; SEQ ID NO. 52) separates between variable regions B and C. An upstream short conserved region of (TTGACAGCTTATCATCGATAAGCT; SEQ ID NO. 53) is located between the vector and region A, and still another, between C and the vector (CAAGACGTAGCCCAGCGCGTCGGCCGGCGAT; SEQ ID NO. 54). Each of the primers used for the isolation of fragments from pBR322 comprised an overhanging portion (Table 2, in bold) encoding the uniform overlapping sequences.

**Table 2:**

| Region | Variable sequence | Length (bps) | Template for PCR Amplification (coresequence) | The primers that were used to create the segment/ SEQ ID NO. |
|---|---|---|---|---|
| A | 1 | 100 | pBR322(322-380) | |
| | 2 | 150 | pBR322(2196-2304) | |
| B | 1 | 200 | pBR322(502-660) | |
| | 2 | 100 | pBR322(1302-1360) | |
| C | 1 | 150 | pBR322(1927-2035) | |
| | 2 | 350 | pBR322(3361-3669) | |
| LINEAR VECTOR | | 1900 | pBR322(2300-4200) | |

The experiment was conducted as follows:

All of fragments and the vector were amplified from the plasmid pBR322 (GenBank accession No. J01749) using the primers described in Table 2, The primers were purchased from Bio-Technology General Ltd. Rehovot, Israel. The linear vector consisted of the part pBR322 that contains the origin of DNA replication (ORI) and the gene for Ampicillin resistance (Amp^{r}). Each of the amplified fragments was produced with the conserved regions at their ends serving as overlaps of 20-30bp of complementarity between neighboring fragments. The conserved regions were then converted into single stranded overhangs ends which, due to their length, are highly specific and cohesive. The conserved overlaps were generated by using primers that contain two essential parts: the first part is a sequence of 20-30 bases at the 5' end of the primer that is complementary to 20-30 bases at the end of another DNA fragment. This part includes two or three dU nucleotides, which replace dT nucleotides. The second part is the 3' end of the primer. It contains a sequence which is complementary to the template DNA and enables the initiation of the PCR process.

A Stratagene-Robocycler gradient 96 thermocycler was used to calibrate the annealing temperatures. A Techne-Genius thermocycler was then used to amplify the various fragments and the linear vector. Taq DNA Polymerase (Gibco-BRL Life-Technologies) and 10x PCR Buffer (MBI) were added together with dNTPs, template and primers to the PCR reaction mixture. Using a preparative 2% TAE agarose gel, we separated the required fragment (or linear vector) from a 400µl reaction volume. Fragments were purified with the QIAquick Gel Extraction kit (QIAGEN), according to manufacturer instructions. Pharmacia-Biotech Ultrospec 2000 spectrophotometer was used to determine the DNA concentration of the fragments and of the vector.

The overhangs of the linear vector and of the DNA fragments for the construction of the inserts were exposed as described above. Immediately after heating, the DNA fragments were allowed to anneal by slowly cooling the mixtures from 70°C to 37°C, in 20mM Tris-HCl pH 8.4, 50mM KCl, 1.5mM MgCl₂.

For transformation, with A BioRad E. coli pulser bacteria ElectroMAX DH10B cells from Gibco-BRL Life-Technologies were used. All the electroporation processes were performed in duplicates, transformed cells were transferred to 1ml of LB and grown with shaking at 37°C for 1hr. In each case, before plating one of each of the duplicates was concentrated and plated, and the other was diluted (up to 1/100) and plated on LB-agar plates containing Ampicillin These plates were incubated in a 37°C incubator overnight at which point the colonies were counted and examined.

Chosen colonies were incubated over-night in a 37°C shaker in 1.5ml LB medium containing Ampicillin. Cells were harvested by centrifugation, and lysed using the first three solutions from High Pure Plasmid Isolation Kit (Boehringer-Mannheim) or from Concert Rapid Plasmid Purification Systems (Gibco-BRL, Life Technologies). Plasmids to be sequenced were treated following manufacturer's instructions. Those plasmids that were chosen for restriction analysis were ethanol precipitated as follow: After the addition of the three solutions according to manufacturer's instructions, the lysates were centrifuged at top speed in an Eppendorf tabletop centrifuge for 10 minutes. The supernatants were removed into new tubes, and the DNA was precipitated in 70% isopropanol. After incubation at room temperature for 10min, the samples were centrifuged at top speed in an Eppendorf tabletop centrifuge for 10min. The pellets were washed with 70% ethanol, dried, and resuspended. Restriction enzyme analyses were performed according to known procedures.

The names of the fragments used in this study and the primers used for their PCR analysis are listed in Table 2. The combinatorial constructs were analyzed by: a) PCR analysis using primers with sequences residing on the vector and conserved regions. The primers used were: AGGGCGACACGGAAATGTTG (SEQ ID NO. 59) and AAGCGGAAGAGCGCCTGATG (SEQ ID NO. 60). PCR was performed in Techne-Genius thermocycler at an annealing temperature of 66°C. b) Restriction enzyme analysis was performed using EarI (purchased from NEB) which has two restriction sites flanking the combinatorial insert was performed. Further restriction enzyme analysis was done with PstI (purchased from NEB) and SphI (purchased from Boehringer-Mannheim). Both PCR and restriction enzyme digestion generated fragments were separated on TAE agarose gels for length determination. c) The same primers used for PCR analysis were also used for sequence determination of the combinatorial construct.

The plasmid library that was generated contains all the optional chimeric products that can be obtained, that is eight different types of constructs, different in length and content (Table 3). 156 colonies were analyzed in which all types of chimeric products were present at a random distribution, as indicated by the different lengths of the amplicons and further verified by restriction analysis of some colonies and further verification by sequence analysis.

**Table 3:**

| Construct conformation | Overall length | Number of colonies |
|---|---|---|
| A1-B1-C1 | 350 | 5 |
| A2-B1-C1 | 400 | 13 |
| A1-B2-C1 | 450 | 21 |
| A2-B2-C1 | 500 | 31 |
| A1-B1-C2 | 550 | 10 |
| A2-B1-C2 | 600 | 12 |
| A1-B2-C2 | 650 | 27 |
| A2-B2-C2 | 700 | 37 |

### EXAMPLE 3. CREATION OF A CHIMERIC CHITINASE A /CHITOBIASE LIBRARY

Chitinase A and Chitobiase from *Serratia marcescens* are two distinct enzymes that belong to the tim-barrel super family. They hydrolyze either polysaccharides or disaccharides respectively.

The tim-barrel cores of both enzymes share distinct structural similarities but are not similar in terms of amino acid sequence. The three dimensional structure of chitinase (PDB code: 1EDQ) and that of chitobiase (PDB code: 1QBA) have been downloaded into ProSUP online protein surposition alignment package in order to detect the related structures. The computer analysis shows that both structures share tertiary structure similarity due to distinct similar secondary structures. These structures are either beta sheets or alpha helices found within the tim-barrel core of both enzymes. One relatively "safe" attitude to search for new traits and improved performance of chitinase is to maintain all the secondary structures of chitinase and replace the less structured portions with varied combinations of the relative sequences from chitobiase. In the present example a more risky approach is taken: The beta sheets of chitinase are maintained while other domains, including alpha helices are being replaced in a combinatorial way. To do so, a set of chitinase, and a set of chitobiase DNA segments have been PCR generated. The various segments were created such that the first segment in both sets start just upstream of the coding region of the genes and end at the first beta sheet of the tim barrel. The variable region of the chitinase segment encodes the region in the gene that is found between the first and second beta sheets. The analogous region of the chitobiase segments encodes the parallel region in chitobiase. The downstream end of the said chitinase segment maintains the DNA sequence of chitinase and therefore the chitinase amino acid sequence as well. In the chitobiase segment, the amino acid sequence of its downstream end is modified such that it is homologous to that of the chitinase. It therefore also encodes the sequence of the chitinase beta sheet.

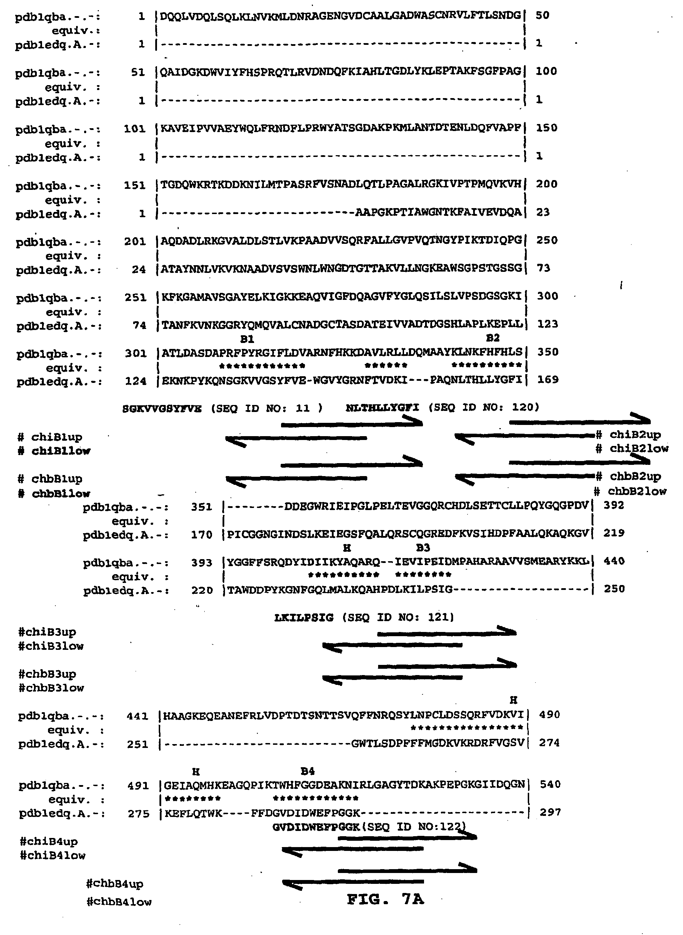

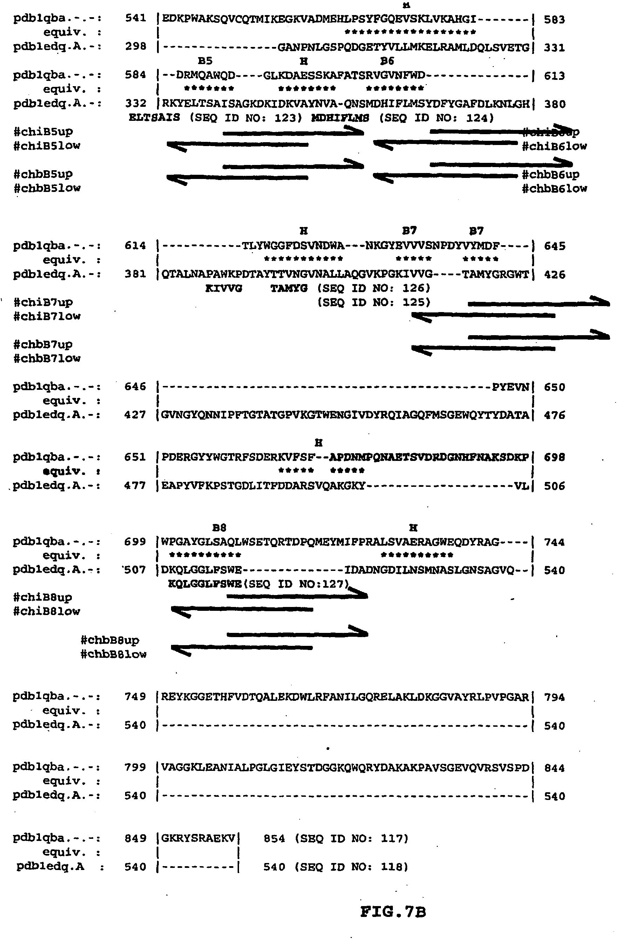

The upstream ends of the second segment in both sets are also homologous to the same sequence that encodes the first chitinase beta sheet. The downstream ends of these segments are homologous to the sequence that encodes the next beta sheet of chitinase while in-between, the variable region of the chitinase segment encodes the chitinase region and that of the chitobiase encodes the sequence of chitobiase. Likewise, the upstream and downstream regions of the third segment in both sets encode the second and third beta sheets of chitinase, respectively. Again the variable region of the segments encode the chitinase and chitobiase regions that are found between the second and third beta sheet domains of both enzymes, respectively. The same is true for the fourth, fifth, and the rest of the segments of both sets. As in Examples 1 and 2, the overlapping ends of the different segments are then converted into single strand overhangs which are allowed to mix and hybridize with each other to assemble into complete genes with various blends of recombinations. The different procedures are the same as those described in Examples 1 and 2. The distinct primers segments and beta sheet junctions are displayed in Table 4 and Figure 7. The assembled constructs are subcloned into pQE30 bacterial expression vector and the primary analysis of chitinase function is carried out in a colorimetric X-NAG colony assay.

**Table 4. Primers used to generate chitinase and chitoblase segments (SEQ ID NOS 71-102, RESPECTIVELY)**

| **sequence** | **Consensus junction** | **common beta-sheet** | **PCR template** | **direction** |
|---|---|---|---|---|
| ##chiB1up | AAAGTGGTCGGUTCTTATTUCGTCGAG-TGGGGCGTTTACGGGCGCAATTTCACCGTCGAC | beta 1 | chitinase | forward |
| ## chiB1low | AAATAAGAACCGACCACTTU-GCCGGAGTTCTGTTTATACGGTTTATTCTTTTCCAGCAGCG | beta 1 | chitinase | reverse |
| # chbB1up | AAAGTGGTCGGUTCTTATTUCGTCGAG-GTGGCGCGCAACTTCCATAAGAAGGACGCGGTGC'£GCGTCTG | beta 1 | chitobiase | forward |
| # chbB1low | AAATAAGAACCGACCACTTU-CGCATCGCTGGCGTCCAGCGTGGCGATCTTGCCGCTGCCGTC | beta 1 | chitobiase | reverse |
| ## chiB2up | AACCTGACCCACCUGCTGTACGGCTTTAUC-CCGATCTGCGGCGGCAATGGCATCAACGACAGC | beta 2 | chitinase | forward |
| ## chiB2low | ATAAAGCCGTACAGCAGGUGGGTCAGGTU-TTGCGCCGGGATCTTGTCGACGGTGAAATTGCGC | beta 2 | chitinase | reverse |
| ## chbB2up | AACCTGACCCACCUGCTGTACGGCTTTAUC-GATGACGAAGGCTGGCGCATCGAGATCCCCGGCTTGC | beta 2 | chitobiase | forward |
| # chbB2low | ATAAAGCCGTACAGCAGGUGGGTCAGGTU-GTAAGCCGCCATCTGATCCAGCAGACGCAGCACCGCG | beta 2 | chitobiase | reverse |
| # chiB3up | AAAATCCUGCCGTCGATCGGU-GGCTGGACGCTGTCCGACCCGTTCTTCTTCATGGGC | beta 3 | chitinase | forward |
| # chiB31ow | ACCGATCGACGGCAGGATTTUCAG-GTCAGGATGCGCCTGCTTCAGCGCCATCAGCTG | beta 3 | chitinase | reverse |
| #chbB3up | AAAATCCUGCCGTCGATCGGU-ATGCCGGCGCACGCGCGCGCCGCGGTGGTTTCGATGGAAGC | beta 3 | chitobiase | forward |
| #chbB31ow | ACCGATCGACGGCAGGATTTUCAG-CTGGCGCGCCTGGGCGTATTTGATGATGTCGATGTAGTC | beta 3 | chitobiase | reverse |
| #chiB4up | ATATCGACTGGGAGTTCCCGGGCGGUAAA-GGCGCCAACCCTAACCTGGGCAGCCCGCAAGACGGGGAAAC | beta 4 | chitinase | forward |
| # chiB41ow | ACCGCCCGGGAACTCCCAGTCGATAUCCACGCC-GAACTCTTTCACCGAACCGACGAAGCGATCGCGCTTCACC | beta 4 | chitinase | reverse |
| #chbB4up | ATATCGACTGGGAGTTCCCGGGCGGUAAA-CGCCTGGGCGCCGOCTATACCGACAAGGCGAAACCGGAA | beta 4 | chitobiase | forward |
| #chbB41ow | ACCGCCCGGGAACTCCCAGTCGATAUCCACGCC-CTTGATCGGCTGCCCGGCTTCTTTATGCATCTGG | beta 4 | chitobiase | reverse |
| #chiB5up | AGCTGACCTCCGCCATCAGU-GCCGGTAAGGACAAGATCGACAAGGTGGCTTAC | beta 5 | chitinase | forward |
| #chiB51ow | ACTGATGGCGGAGGTCAGCUC-ATACTTGCGGCCGGTTTCCGCCGACAGCTGATCC | beta 5 | chitinase | reverse |
| #chbB5up | AGCTGACCTCCGCCATCAGU-GGCCTGAAAGACGCCGAAAGTTCGAAGGCGTTC | beta 5 | chitobiase | forward |
| #chbB5low | ACTGATGGCGGAGGTCAGCUC-ATCGATGCCGTGCGCCTTCACCAGCTTGCTGAC | beta 5 | chitobiase | reverse |
| #chiB6up | ATGGATCACAUCTTCCTGATGAGU-TACGACTTCTATGGCGCGTTCGATCTGAAGAACCTGGGGCATCAGACC | beta 6 | chitinase | forward |
| #chiB6low | ACTCATCAGGAAGAUGTGATCCAU-CGAGTTCTGCGCAACGTTGTAAGCCACCTTGTCGATCTTGTCCTTAC | beta 6 | chitinase | reverse |
| #chbB6up | ATGGATCACAUCTTCCTGATGAGU-ACCCTGTATTGGGGCGGTTTCGACAGCGTTAAC | beta 6 | chitobiase | forward |
| #chbB6low | ACTCATCAGGAAGAUGTGATCCAU-CGAGGTGGCGAACGCCTTCGAACTTTCGGCGTC | beta 6 | chitobiase | reverse |
| #chiB7up | ATCGTGGUCGGCACCGCCATGTATGGU-CGCGGCTGGACCGGGGTGAACGGCTACCAGAACAACATTCCGTTC | beta 7 | chitinase | forward |
| #chiB7low | ATACATGGCGGUGCCGACCACGATCTU-GCCCGGCTTGACGCCCTGCGCCAGCAGCGCATTGACGCCGTTCAC | beta 7 | chitinase | reverse |
| #chbB7up | ATCGTGGUCGGCACCGCCATGTATGGU-CCTTACGAGGTGAATCCGGACGAGCGCGGTTACTACTG | beta 7 | chitobiase | forward |
| #chbB7low | ATACATGGCGGUGCCGACCACGATCTU-ATACCCTTTATTGGCCCAGTCGTTAACGCTGTCGAAACC | beta 7 | chitobiase | reverse |
| #chiB8up | AAGCAGCUGGGCGGCCTGTTCTCCUGGGAG-ATCGACGCGGATAACGGCGATATTCTCAACAGCATG | beta 8 | chitinase | forward |
| #chiB81ow | AGGAGAACAGGCCGCCCAGCTGCTT-ATCCAGCACGTACTTGCCTTTGGCCTGCACCGAGC | beta 8 | chitinase | reverse |
| #chbB8up | AAGCAGCUGGGCGGCCTGTTCTCCUGGGAG-TGGAGCGAAACCCAGCGCACCGATCCGCAGATGGAATA | beta 8 | chitobiase | forward |
| #chbBB1ow | AGGAGAACAGGCCGCCCAGCTGCTT-CCACGGCTTGTCGCTTTTGGCGTTGAAGTGGTTGCCGTCGCGGTC | beta 8 | chitobiase | reverse |

## Claims

1. A method for generating divergent libraries of recombinant chimeric enzymes, said method comprising:
(a) identifying a plurality of conserved amino acid sequences in a plurality of related enzymes;
(b) selecting a plurality of consensus amino acid sequences as a backbone corresponding to said conserved amino acid sequences to serve as sites of recombination and a backbone of enzymes created and selecting a plurality of variable regions corresponding to non-conserved amino acid sequences in said plurality of related enzymes;
(c) generating a plurality of partially overlapping polynucleotides comprising a nucleic acid sequence encoding the consensus amino acid sequences of (b), wherein each polynucleotide comprises: (i) at least one terminal oligonucleotide sequence complementary to a terminal oligonucleotide sequence of at least one other polynucleotide, and wherein at least one terminal sequence at the terminus of each polynucleotide encodes an intact consensus amino acid of (b); and (ii) a polynucleotide sequence encoding variable, non-conserved amino acid sequence selected from any of the plurality of related enzymes of (b);
(d) inducing recombination between the plurality of said partially overlapping polynucleotides of (c) to produce divergent libraries of chimeric polynucleotides wherein the recombinations intentionally take place between the sequences that correspond to the full length consensus amino acids;
(e) transfecting a plurality of host cells with the chimeric polynucleotides of (d) to produce divergent libraries of cloned cell lines capable of expressing one of the recombinant chimeric enzymes; and optionally
(f) recovering recombinant chimeric enzymes from the cloned cell lines of (e).

2. The method of claim 1, wherein the consensus amino acid sequence is a segment of 3 to 30 amino acids, that is conserved in the plurality of related enzymes.

3. The method of claim 1, comprising substituting amino acid residues having similar side chains including aliphatic, aliphatic-hydroxyl, amide, aromatic, basic or sulfur-containing side chains.

4. The method of claim 1, wherein the plurality of overlapping polynucleotides comprise variable sequences having less than 70% or less than 50% or less than 30% or 10% sequence homology.

5. The method of claim 1, wherein recombination occurs in vitro.

6. The method of claim 1, wherein the plurality of overlapping polynucleotides is amplified prior to recombination.

7. The method of claim 1, wherein recombination between the plurality of overlapping polynucleotides takes place in the presence of a plurality of vector fragments, wherein the sequence at each end of a vector fragment is complementary to at least one terminal oligonucleotide sequence of at least one of said overlapping polynucleotides.

8. The method of claim 1, wherein the library of chimeric polynucleotides is ligated into vectors prior to transformation into a plurality of host cells.

9. The method of claim 1, using at least one cloned cell line having a specific enzymatic activity.

10. The method of claim 1, using at least one recovered recombinant chimeric protein having a specific enzymatic activity.

11. The method of claim 8, wherein each vector is used with at least one further component selected from the group consisting of restriction enzyme site, selection marker gene, an element necessary for propagation, an element necessary for maintenance, an element necessary for expression, and an element capable of regulating production of a detectable enzymatic activity.

12. The method of claim 1, further comprising adding the enzyme Uracil DNA Glycosylase (UDG) at the recombination step.

13. The method of claim 12, further comprising adding N, N, dimethylethylenediamine (DMED).

14. The method of claim 13, further comprising adding the Ezyme ligase prior to the recombination step.

## Patentansprüche

1. Verfahren zur Erzeugung divergenter Bibliotheken von rekombinanten chimären Enzymen, wobei das Verfahren Folgendes umfasst:
(a) Identifizierung einer Vielzahl von konservierten Aminosäuresequenzen in einer Vielzahl von verbundenen Enzymen;
(b) Auswahl einer Vielzahl von Konsensaminosäuresequenzen als ein Rückgrat, entsprechend den konservierten Aminosäuresequenzen, um als Rekombinationsstellen zu dienen und ein Rückgrat von erzeugten Enzymen und auswählend eine Vielzahl von variablen Bereichen, entsprechend nicht konservierten Aminosäuresequenzen in der Vielzahl von verbundenen Enzymen;
(c) Erzeugung einer Vielzahl von teilweise überlappenden Polynukleotiden, umfassend eine Nukleinsäuresequenz, die die Konsensaminosäuresequenzen von (b) kodiert, wobei jedes Polynukleotid Folgendes umfasst: (i) mindestens eine endständige Oligonukleotidsequenz, die eine endständige Oligonukleoidsequenz von mindestens einem anderen Polynukleotid ergänzt, und wobei mindestens eine endständige Sequenz am Terminus jedes Polynukleotids eine intakte Konsensaminosäure von (b) kodiert; und(ii) eine Polynukleotidsequenz, die variable, nicht konservierte Aminosäuresequenzen, ausgewählt aus einer der Vielzahl von verbundenen Enzymen von (b), kodiert;
(d) Induktion der Rekombination zwischen der Vielzahl von teilweise überlappenden Polynukleotiden von (c), um divergierende Bibliotheken von chimären Polynukleotiden zu erzeugen, wobei die Rekombinationen absichtlich zwischen den Sequenzen stattfinden, die der vollen Länger de Konsensaminosäuren entsprechen;
(e) Transfektion einer Vielzahl von Wirtszellen mit den chimären Polynukleotiden von (d), um divergente Bibliotheken von geklonten Zelllinien zu erzeugen, die dazu in der Lage sind, eines der rekombinanten Enzyme auszudrücken; und wahlweise
(f) Wiedergewinnung von rekombinanten chimären Enzymen von den geklonten Zelllinien von (e).

2. Verfahren nach Anspruch 1, wobei die Konsensaminosäuresequenz ein Segment von 3 bis 30 Aminosäuren ist, das in der Vielzahl von verbundenen Enzymen konserviert ist.

3. Verfahren nach Anspruch 1, umfassend den Ersatz von Aminosäurerückständen mit ähnlichen Seitenketten, einschließend aliphatische, aliphatische Hydroxyl-, Amid-, aromatische, basische oder Schwefel enthaltende Seitenketten.

4. Verfahren nach Anspruch 1, wobei die Vielzahl von überlappenden Polynukleotiden variable Sequenzen mit weniger als 70 % oder weniger als 50 % oder weniger als 30 % oder 10 % Sequenzhomologie aufweist.

5. Verfahren nach Anspruch 1, wobei die Rekombination in vitro erfolgt.

6. Verfahren nach Anspruch 1, wobei die Vielzahl von überlappenden Polynukleotiden von der Rekombination erweitert wird.

7. Verfahren nach Anspruch 1, wobei die Rekombination zwischen der Vielzahl von überlappenden Polynukleotiden in Anwesenheit einer Vielzahl von Vektorfragmenten erfolgt, wobei die Sequenz an jedem Ende eines Vektorfragments mindestens einer endständigen Oligonukleotidsequenz mindestens eines der überlappenden Polynukleotide entspricht.

8. Verfahren nach Anspruch 1, wobei die Bibliothek von chimären Polynukleotiden vor der Transformation in eine Veilzahl von Wirtszellen in Vektoren ligiert wird.

9. Verfahren nach Anspruch 1, verwendend mindestens eine geklonte Zelllinie mit einer spezifischen enzymatischen Aktivität.

10. Verfahren nach Anspruch 1, verwendend mindestens ein wiedergewonnenes rekombinantes chimäres Protein mit einer spezifischen enzymatischen Aktivität.

11. Verfahren nach Anspruch 8, wobei jeder Vektor mit mindestens einer weiteren Komponente verwendet wird, ausgewählt aus der Gruppe umfassend Restriktionsenzymstelle, Selektionsmarkergen, ein Element, das zur Fortpflanzung erforderlich ist, ein Element, das zur Aufrechterhaltung erforderlich ist, ein Element, das zum Ausdruck erforderlich ist, und ein Element, das dazu in der Lage ist, die Erzeugung einer nachweisbaren enzymatischen Aktivität zu regulieren.

12. Verfahren nach Anspruch 1, weiter umfassend den Zusatz des Enzyms Uracil-DNA-Glycosylase (UDG) im Rekombinationsschritt.

13. Verfahren nach Anspruch 12, weiter umfassend den Zusatz von N, N, dimethylethylendiamin (DMED).

14. Verfahren nach Anspruch 13, weiter umfassend den Zusatz des Enzyms Ligase vor dem Rekombinationsschritt.

## Revendications

1. Procédé de production de bibliothèques diversifiées d'enzymes chimères recombinantes, ledit procédé consistant à :
a) identifier une pluralité de séquences conservées d'acides aminés dans une pluralité d'enzymes associées ;
b) sélectionner une pluralité de séquences consensus d'acides aminés comme armature correspondant auxdites séquences conservées d'acides aminés destinées à servir de sites de recombinaison et comme armature des enzymes créées, et sélectionner une pluralité de régions variables correspondant à des séquences non conservées d'acides aminés dans ladite pluralité d'enzymes associées ;
c) produire une pluralité de polynucléotides se chevauchant partiellement comprenant une séquence d'acide nucléique codant pour les séquences consensus d'acides aminés du point b), dans laquelle chaque polynucléotide comprend : i) au moins une séquence oligonucléotidique terminale complémentaire d'une séquence oligonucléotidique terminale d'au moins un autre polynucléotide et dans laquelle au moins une séquence terminale située sur la terminaison de chaque polynucléotide code pour un acide aminé consensus intact du point b) ; et ii) une séquence polynucléotidique codant pour une séquence variable et non conservée d'acides aminés sélectionnée à partir de l'une quelconque de la pluralité d'enzymes associées du point b) ;
d) induire une recombinaison entre la pluralité desdits polynucléotides se chevauchant partiellement du point c) pour produire des bibliothèques diversifiées de polynucléotides chimères, dans laquelle les recombinaisons se produisent intentionnellement entre les séquences qui correspondent aux acides aminés consensus de pleine longueur ;
e) transfecter une pluralité de cellules hôtes avec les polynucléotides chimères du point d) pour produire des bibliothèques diversifiées de lignées cellulaires clonées aptes à exprimer une des enzymes chimères recombinantes ; et facultativement
f) récupérer des enzymes chimères recombinantes à partir des lignées cellulaires clonées du point e).

2. Procédé selon la revendication 1, dans lequel la séquence consensus d'acides aminés est un segment de 3 à 30 acides aminés qui est conservé dans la pluralité d'enzymes associées.

3. Procédé selon la revendication 1, consistant à substituer des résidus d'acides aminés ayant des chaînes latérales similaires, y compris des chaînes latérales aliphatiques, aliphatiques-hydroxyles, amides, aromatiques, basiques ou soufrées.

4. Procédé selon la revendication 1, dans lequel la pluralité de polynucléotides se chevauchant partiellement comprennent des séquences variables ayant moins de 70% ou moins de 50% ou moins de 30% ou moins de 10% d'homologie de séquence.

5. Procédé selon la revendication 1, dans lequel la recombinaison se produit in vitro.

6. Procédé selon la revendication 1, dans lequel la pluralité de polynucléotides se chevauchant est amplifiée avant d'être recombinée.

7. Procédé selon la revendication 1, dans lequel la recombinaison entre la pluralité de polynucléotides se chevauchant a lieu en présence d'une pluralité de fragments de vecteurs, la séquence située à chaque extrémité d'un fragment de vecteur étant complémentaire d'au moins une séquence oligonucléotidique terminale d'au moins un desdits polynucléotides se chevauchant.

8. Procédé selon la revendication 1, dans lequel la bibliothèque de polynucléotides chimères est ligaturée en vecteurs avant d'être transformée en une pluralité de cellules hôtes.

9. Procédé selon la revendication 1, utilisant au moins une lignée cellulaire clonée ayant une activité enzymatique spécifique.

10. Procédé selon la revendication 1, utilisant au moins une protéine chimère recombinante récupérée ayant une activité enzymatique spécifique.

11. Procédé selon la revendication 8, dans lequel chaque vecteur est utilisé avec au moins un autre composant choisi dans le groupe constitué par un site d'enzyme de restriction, un gène marqueur de sélection, un élément nécessaire à la propagation, un élément nécessaire au maintien, un élément nécessaire à l'expression, et un élément apte à réguler la production d'une activité enzymatique détectable.

12. Procédé selon la revendication 1, consistant en outre à ajouter l'enzyme uracile ADN glycosylase (UDG) lors de l'étape de recombinaison.

13. Procédé selon la revendication 12, consistant en outre à ajouter de la N, N, diméthyl-éthylènediamine (DMED).

14. Procédé selon la revendication 13, consistant en outre à ajouter l'enzyme ligase avant l'étape de recombinaison.
